# EUROPEAN PATENT SPECIFICATION

(11) **EP 3 405 209 B1**
(45) Date of publication and mention of the grant of the patent: **26.05.2021**
(21) Application number: 17740910.9
(22) Date of filing: 19.01.2017
(51) Int. Cl.: A61K 38/04, A61K 38/19, C07K 14/52, C07K 14/525, C07K 14/555, C07K 14/57, A61P 35/00, A61P 9/10

(54) **NOVEL BIOMOLECULE CONJUGATES AND USES THEREFOR**
NEUARTIGE BIOMOLEKÜLKONJUGATE UND VERWENDUNGEN DAVON
NOUVEAUX CONJUGUÉS BIOMOLÉCULAIRES ET LEURS UTILISATIONS

(30) Priority: 19.01.2016 US 201662280351 P
(43) Date of publication of application: 28.11.2018
(73) Proprietor: The University Of Western Australia, Crawley, WA 6009 (AU); Sanford Burnham Prebys Medical Discovery Institute, La Jolla, CA 92037 (US)
(72) Inventor: HAMZAH, Juliana Binti, Coolbellup Western Australia 6163 (AU); GANSS, Ruth Annelore, Nedlands Western Australia 6009 (AU); RUOSLAHTI, Erkki, Rancho Santa Fe California 92067 (US); BILIRAN JR., Hector R., New Orleans Louisiana 70115 (US)
(74) Representative: Brann AB
(86) International application number: PCT/AU2017/050037
(87) International publication number: WO 2017/124147

(56) References cited:
- WO-A1-97/10507
- WO-A1-99/13329
- WO-A2-00/42973
- WO-A2-2011/106788
- WO-A2-2016/187508
- CN-B- 101 302 255
- US-A1- 2013 115 167
- YAO H ET AL: "Enhanced effect of soluble transforming growth factor-[beta] receptor II and IFN-[gamma] fusion protein in reversing hepatic", EUROPEAN JOURNAL OF MEDICAL RESEARCH, BIOMED CENTRAL LTD, LONDON, UK, vol. 15, no. 4, 8 April 2010 (2010-04-08), page 152, XP021132150, ISSN: 2047-783X, DOI: 10.1186/2047-783X-15-4-152
- MCLAREN J E ET AL: "Interferon gamma: A master regulator of atherosclerosis", CYTOKINE AND GROWTH FACTOR REVIEWS, ELSEVIER LTD, GB, vol. 20, no. 2, 1 April 2009 (2009-04-01), pages 125-135, XP026036633, ISSN: 1359-6101, DOI: 10.1016/J.CYTOGFR.2008.11.003 [retrieved on 2008-11-28]
- HU SHENG-JUAN ET AL: "Purification of a Pd20-TNF[alpha] fusion protein that prevents liver metastasis of gastric ca", TUMOR BIOLOGY, KARGER, BASEL, CH, vol. 35, no. 8, 2 May 2014 (2014-05-02), pages 7523-7529, XP035387711, ISSN: 1010-4283, DOI: 10.1007/S13277-014-1957-2 [retrieved on 2014-05-02]

## Description

### FIELD OF THE INVENTION

The present invention relates generally to biomolecule conjugates comprising a cytokine, typically an immunopotentiating cytokine, such as tumour necrosis factor α (TNFα), and a peptide comprising or consisting of the sequence CSGRRSSKC (SEQ ID NO:1), and to uses of this conjugate for imaging and treatment of solid tumours, atherosclerotic tissue and fibrotic tissue. The present invention also relates to the use of a peptide comprising or consisting of the sequence of SEQ ID NO: 1, optionally linked to a detectable agent and/or a carrier, in the detection and/or localisation of tumour, atherosclerotic and fibrotic tissue.

### BACKGROUND OF THE INVENTION

In comparison to healthy, non-cancerous tissue, solid tumours typically possess a number of structural features that can limit access of drugs to the tumour and to the cancerous cells. These include an abnormal vasculature and an abnormally dense extracellular matrix (ECM). The abnormal, compressed vasculature results in poor blood supply into and through the tumour tissue, meaning most drug transport is by diffusion, however this is significantly hindered by the complex and dense structure of the tumour ECM characterized by elevated levels of collagen and glycosaminoglycans.

As a result of increased ECM density, solid tumours are stiffer than normal tissue and palpable as hard nodules. For example, breast cancer tissue is up to 10 times stiffer than normal breast tissue, and advanced hepatocellular carcinoma (HCC) is two to five times stiffer than benign tumours or fibrotic liver. Matrix stiffness determines how migrating cells and other circulating reagents enter or exit the tumour. Thus, a dense tumour ECM represents a significant physical barrier that isolates tumours from their surroundings and prevents access to anti-cancer drugs.

Local injection of ECM-degrading proteases such as collagenase, hyaluronidase, matrix metalloproteinases, delaxin and decorin into solid tumours have been shown to reduce ECM content and enhance drug uptake. However, currently none of these proteases, including pegylated recombinant hyaluronidase (PEGPH20) currently in phase I clinical trial, are engineered to specifically target and degrade tumour ECM. Hence, their applications are less effective and toxic if administered through systemic circulation. Additionally, the application of multiple proteases in combination may be required to sufficiently reduce matrix content and stiffness to allow drug access, however such combinatorial use of proteases is not viable as this would further elevate systemic toxicity.

Cancer mortality and morbidity can be improved by effective imaging of tumours enabling effective screening, diagnosis and identification of cancerous cells and of tumours. However a dense tumour ECM also acts as a barrier to cancer diagnostic and imaging agents.

Targeting tumour ECM is a promising but so far under-explored approach to improve clinical cancer management. There exists a clear need for improved means of targeting tumour ECM, to degrade or destroy the ECM in order to improve tumour accessibility for drug penetration, and also to prove access to tumours by imaging agents thereby improving cancer detection.

ECM remodelling also contributes significantly to fibrosis. Fibrosis is the abnormal accumulation of fibrous (or scar) tissue that can occur as a part of the wound-healing process in damaged tissue. Liver (hepatic) fibrosis, for example, occurs as a part of the wound-healing response to chronic liver injury. Liver fibrosis is characterized by the accumulation of extracellular matrix that can be distinguished qualitatively from that in normal liver. Hepatic fibrosis if untreated can progress to cirrhosis, hepatocellular carcinoma, liver failure, and death.

Disclosed herein are novel biomolecule conjugates comprising a cytokine, such as the immunopotentiating cytokine TNFα, and a peptide containing the motif CSG, and uses of said conjugates in the treatment and detection of tumours and fibrosis. Fusion proteins involving TNFα have previously been considered for tumour therapy, including TNFα-NGR, currently in clinical trial. However TNFα-NGR targets the tumour vasculature and has no effect on tumour ECM. Thus, the above-noted problems associated with access of drugs to tumours, and thus efficacy of these drugs, remain.

### SUMMARY OF THE INVENTION

As described and exemplified herein, the present inventors have generated novel biomolecule conjugates capable of specifically targeting and degrading ECM, including tumour ECM thereby improving tumour perfusion and circulatory uptake of tumour imaging agents and therapeutic agents. Also described and exemplified herein is the ability of these novel conjugates to target and degrade atherosclerotic plaque ECM.

One aspect of the invention provides a biomolecule conjugate comprising a cytokine and a peptide comprising or consisting of the sequence set forth in SEQ ID NO:1 or a conservative variant thereof.

In a particular embodiment the cytokine is an immunopotentiating cytokine. In an embodiment, the immunopotentiating cytokine is a cytokine that mediates a cellular immune response. Exemplary immunopotentiating cytokines of the invention include, but are not limited to, TNFα, interferon-γ (IFNγ) and interferon-1β (IFN-1β). In exemplary embodiments, the immunopotentiating cytokine is TNFα or IFNγ.

In an embodiment, the cellular immune response may be stimulation of expression and/or secretion of multiple proteases. Accordingly, as described hereinbelow, the present invention provides a biomolecule conjugate comprising a cytokine and a peptide comprising or consisting of the sequence set forth in SEQ ID NO:1 or a conservative variant thereof for use in methods for stimulating the expression and/or secretion of multiple proteases within tissue with an abnormal ECM, typically tumours, atherosclerotic tissue and fibrotic tissue, to thereby induce or promote degradation of the ECM.

In a further embodiment, the peptide comprising or consisting of the sequence set forth in SEQ ID NO:1, or conservative variant thereof, is conjugated to the C-terminal end of the immunopotentiating cytokine. The peptide may be conjugated to the immunopotentiating cytokine via a linker sequence. In exemplary embodiments the linker may comprise one or more, optionally two or more, or three or more, glycine (G) residues.

The biomolecule conjugate may be conjugated or otherwise linked to a carrier, optionally a nanoparticle carrier. In an exemplary embodiment the carrier comprises iron oxide (IO) nanoparticles or micelles.

Also provided is a polynucleotide encoding a biomolecule conjugate of the present invention.

Another aspect of the invention provides a pharmaceutical composition comprising a biomolecule conjugate of the present invention, or a polynucleotide encoding the same, wherein the composition typically further comprises one or more pharmaceutically acceptable carriers, adjuvants and/or excipients. The pharmaceutical composition may further comprise one or more additional therapeutic agents, such as anti-tumorigenic agents, anti-atherosclerotic agents and/or anti-fibrotic agents.

A further aspect of the invention provides a biomolecule conjugate or pharmaceutical composition of the present invention for use in a method for degrading the extracellular matrix (ECM) of tumour, atherosclerotic plaque or fibrotic tissue, comprising exposing the tissue to an effective amount of a biomolecule conjugate or pharmaceutical composition of the present invention.

In an embodiment, the degradation of the ECM results from or is associated with an increase in the expression and/or secretion of two or more proteases within the tissue following exposure to the biomolecule conjugate or pharmaceutical composition. The two or more proteases may comprise matrix metalloproteases, cathepsins, disintegrins and/or ADAM proteases. In an exemplary embodiment the proteases may be selected from two or more of uPA, MMP-2, MMP-3, MMP9, MMP-12, MMP-14, cathepsin B, Cathepsin L and ADAM-9. In an embodiment the expression and/or secretion of uPA, MMP-2, MMP-3, MMP9, MMP-12, MMP-14, cathepsin B, Cathepsin L and ADAM-9 is increased.

Accordingly, a further aspect of the invention provides a biomolecule conjugate or pharmaceutical composition of the present invention for use in a method for increasing the expression and/or secretion of two or more proteases within a tumour, atherosclerotic tissue or fibrotic tissue, the method comprising exposing the tumour or tissue to an effective amount of a biomolecule conjugate or pharmaceutical composition of the present invention.

The two or more proteases may comprise matrix metalloproteases, cathepsins, disintegrins and/or ADAM proteases. In an exemplary embodiment the proteases may be selected from two or more of uPA, MMP-2, MMP-3, MMP9, MMP-12, MMP-14, cathepsin B, Cathepsin L and ADAM-9. In an embodiment the expression and/or secretion of uPA, MMP-2, MMP-3, MMP9, MMP-12, MMP-14, cathepsin B, Cathepsin L and ADAM-9 is increased.

The increased expression and/or secretion of the two or more proteases induces, promotes or results in the degradation of ECM of the tumour, atherosclerotic tissue or fibrotic tissue.

A further aspect of the invention provides a biomolecule conjugate or pharmaceutical composition of the present invention for use in a method for promoting or inducing immune cell infiltration of a tumour, atherosclerotic plaque or fibrotic tissue, comprising exposing the tissue to an effective amount of a biomolecule conjugate or pharmaceutical composition of the present invention.

In an embodiment, the immune cells infiltrating the tumour or fibrotic tissue express and release one or more proteases capable of degrading the tumour or fibrotic tissue ECM. The immune cells may comprise T cells, macrophages and/or neutrophils. In an exemplary embodiment the T cells are CD4⁺ and/or CD8⁺ T cells. In an exemplary embodiment the macrophages or neutrophils are CD11b⁺, CD68⁺ and/or F4/80⁺.

A further aspect of the invention provides a biomolecule conjugate or pharmaceutical composition of the present invention for use in a method for treating a condition associated with abnormal ECM, comprising administering to the subject an effective amount of a biomolecule conjugate or pharmaceutical composition of the present invention.

Typically the condition associated with abnormal ECM is selected from a tumour, atherosclerosis or fibrosis.

A further aspect of the invention provides a biomolecule conjugate or pharmaceutical composition of the present invention for use in a method for treating a solid tumour in a subject, comprising administering to the subject an effective amount of a biomolecule conjugate or pharmaceutical composition of the present invention.

Prior to said treatment the tumour may display resistance to therapy with one or more anti-cancer agents. The one or more anti-cancer agents may comprise chemotherapeutic, immunotherapeutic and/or radiotherapeutic agents.

The conjugate may be administered to the subject in combination with one or more additional anti-cancer agents, typically chemotherapeutic, immunotherapeutic and/or radiotherapeutic agents. The conjugate and the one or more additional anti-cancer agents may be in the same or in different compositions. Thus, the conjugate may be administered to the subject prior to, concomitantly with, or subsequent to the one or more additional anti-cancer agents.

Treatment of the tumour with the conjugate may increase vessel perfusion in the tumour, increasing access of the one or more additional anti-cancer agents to the tumour and cancerous cells therein and thereby improving efficacy of said anti-cancer agents.

Accordingly, a further aspect provides a biomolecule conjugate or pharmaceutical composition of the present invention for use in a method for increasing the sensitivity of a tumour to an anti-cancer agent, the method comprising exposing the tumour to an effective amount of a biomolecule conjugate or pharmaceutical composition of the present invention.

The tumour may be resistant to one or more anti-cancer agents, in the absence of said treatment.

A further aspect of the invention provides a biomolecule conjugate or pharmaceutical composition of the present invention for use in a method for increasing or extending the survival time of a subject having a tumour, the method comprising administering to the subject an effective amount of a biomolecule conjugate or pharmaceutical composition of the present invention.

A further aspect of the invention provides a biomolecule conjugate or pharmaceutical composition of the present invention for use in a method for treating fibrosis in a subject, comprising administering to the subject an effective amount of a biomolecule conjugate or pharmaceutical composition of the present invention.

In exemplary embodiments the fibrosis is liver fibrosis, cardiac fibrosis or vascular fibrosis. The liver fibrosis may be early stage fibrosis, advanced fibrosis or cirrhosis. The cardiac or vascular fibrosis may comprise fibroatheromas or be otherwise associated with atherosclerotic plaques. The fibrosis may be pre-cancerous fibrosis.

The conjugate may be administered to the subject in combination with one or more additional anti-fibrotic agents. The conjugate and the one or more additional anti-fibrotic agents may be in the same or in different compositions. Thus, the conjugate may be administered to the subject prior to, concomitantly with, or subsequent to the one or more additional anti-fibrotic agents.

Treatment of the fibrotic tissue with the conjugate may increase vessel perfusion, increasing access of the one or more additional anti-fibrotic agents to the fibrotic tissue and thereby improving efficacy of said anti-fibrotic agents.

Accordingly, a further aspect provides a biomolecule conjugate or pharmaceutical composition of the present invention for use in a method for increasing the sensitivity of fibrotic tissue to an anti-fibrotic agent, the method comprising exposing the fibrotic tissue to an effective amount of a biomolecule conjugate or pharmaceutical composition of the present invention.

A further aspect of the invention provides a biomolecule conjugate or pharmaceutical composition of the present invention for use in a method for treating or preventing atherosclerosis in a subject, the method comprising administering to the subject an effective amount of a biomolecule conjugate or pharmaceutical composition of the present invention.

The treating or preventing atherosclerosis may comprise reducing atherosclerotic plaque formation. The treating or preventing atherosclerosis may comprise modulating blood cholesterol levels. In an embodiment, plasma levels of HDL may be increased and/or plasma levels of LDL may be decreased relative to the levels observed in the absence of administration of the biomolecule conjugate or pharmaceutical composition.

The conjugate may be administered to the subject in combination with one or more additional anti-atherosclerotic agents. The conjugate and the one or more additional anti-atherosclerotic agents may be in the same or in different compositions. Thus, the conjugate may be administered to the subject prior to, concomitantly with, or subsequent to the one or more additional anti-atherosclerotic agents.

Treatment of the atherosclerotic plaque tissue with the conjugate may increase perfusion, increasing access of the one or more additional anti-atherosclerotic agents to the plaque tissue and thereby improving efficacy of said anti-atherosclerotic agents.

Accordingly, another aspect of the invention provides a biomolecule conjugate or pharmaceutical composition of the present invention for use in a method for increasing the sensitivity of an atherosclerotic plaque to an anti-atherosclerotic agent, the method comprising exposing the plaque to an effective amount of a biomolecule conjugate or pharmaceutical composition of the present invention.

A further aspect of the invention provides a biomolecule conjugate or pharmaceutical composition of the present invention for use in a method for modulating blood cholesterol levels, the method comprising administering to a subject in need thereof an effective amount of a biomolecule conjugate or pharmaceutical composition of the present invention.

The modulation of blood cholesterol levels typically comprises increasing plasma levels of HDL and/or decreasing plasma levels of LDL, relative to the levels observed in the absence of administration of the biomolecule conjugate or pharmaceutical composition.

A further aspect of the invention provides a biomolecule conjugate or pharmaceutical composition of the present invention for use in a method for identifying, imaging or localizing cancerous cells, tumours, atherosclerotic plaque and fibrotic tissue in a subject, comprising administering to the subject a biomolecule conjugate or pharmaceutical composition of the present invention in combination with an agent for imaging or visualising a tumour, cancerous cells, atherosclerotic plaque or fibrotic tissue.

The conjugate and the imaging agent may be in the same or in different compositions. Thus, the conjugate may be administered to the subject prior to, concomitantly with, or subsequent to the imaging agent.

Accordingly, embodiments of the present invention provide means for detecting a tumour or cancerous cells in a subject, wherein a biomolecule conjugate or pharmaceutical composition of the present invention is administered in combination with an agent for imaging or visualising a tissue or cells. Embodiments also provide means for detecting fibrosis in a subject, wherein a biomolecule conjugate or pharmaceutical composition of the present invention is administered in combination with an agent for imaging or visualising tissue or cells. Embodiments also provide means for detecting atherosclerosis or an atherosclerotic plaque in a subject, wherein a biomolecule conjugate or pharmaceutical composition of the present invention is administered in combination with an agent for imaging or visualising tissue or cells.

Also provided is the use of a cytokine, typically an immunopotentiating cytokine such as TNFα, and a peptide comprising or consisting of the sequence set forth in SEQ ID NO: 1, typically as a biomolecule conjugate as disclosed herein, for the manufacture of a medicament: for degrading tumour ECM; for degrading fibrotic ECM; for degrading atherosclerotic plaque ECM; for promoting or inducing immune cell infiltration of tumours, atherosclerotic or fibrotic tissue; for treating tumours and fibrosis; for treating or preventing atherosclerosis; or for detecting, localising or imaging a tumour, cancerous cells, atherosclerotic or fibrotic tissue.

As described and exemplified herein, the present inventors have also elucidated, for the first time, the ability of a peptide comprising or consisting of the sequence set forth in SEQ ID NO: 1 to target the ECM of a variety of tumour types, and atherosclerotic and fibrotic tissue.

Accordingly, an aspect of the invention provides a peptide comprising or consisting of the sequence set forth in SEQ ID NO:1 for use in the detection and/or localisation of tumour, atherosclerosis or fibrosis in a subject or a tissue sample obtained from a subject.

The peptide may be conjugated or otherwise linked to a detectable label or agent and/or a carrier. The carrier may be capable of detection and/or localisation by imaging, such as, for example, a nanoparticle-based carrier. The nanoparticle carrier may comprise, for example, iron oxide (IO) micelles.

It is also described herein a peptide comprising or consisting of the sequence set forth in SEQ ID NO: 1 for use for targeting tumour, atherosclerotic or fibrotic tissue.

The tumour may be a lung tumour, such as small cell lung cancer or non-small cell lung cancer; a pancreatic tumour, such as an insulinoma; a bladder tumour; a kidney tumour; a brain tumour, such as a glioblastoma or medulloblastoma; a neuroblastoma; a head and neck tumour; a thyroid tumour; a breast carcinoma; a cervical tumour; a prostate tumour; a testicular tumour; an ovarian tumour; an endometrial tumour; a rectal and colorectal tumour; a stomach tumour; an esophageal tumour; a skin tumour, such as a melanoma or squamous cell carcinoma; an oral tumour including squamous cell carcinoma; a liver tumour, including human hepatocellular carcinona (HCC); a lymphomas; a sarcomas, including osteosarcoma, liposarcoma and fibrosarcoma.

The fibrosis may be, for example, liver fibrosis, cardiac fibrosis, vascular fibrosis, kidney fibrosis, lung fibrosis or skin fibrosis. The fibrosis may be pre-cancerous fibrosis.

Typically the atherosclerotic tissue is a fibroatheroma or atherosclerotic plaque.

Also provided herein are imaging agents that comprise a peptide linked to a detectable label or agent, wherein the peptide comprises or consists of sequence set forth in SEQ ID NO: 1.

The peptide may be conjugated or otherwise linked to a detectable label or agent and/or a carrier.

An aspect of the invention also provides a peptide comprising or consisting of the sequence set forth in SEQ ID NO: 1 for use in a method for detecting and/or localising tumour, atherosclerotic or fibrotic tissue, comprising exposing tissue, or a biological sample comprising tissue, to a peptide comprising or consisting of the sequence set forth in SEQ ID NO: 1. A further aspect provides a peptide comprising or consisting of the sequence set forth in SEQ ID NO: 1 for use in a method for targeting tumour, atherosclerotic or fibrotic tissue, comprising exposing the tissue to a peptide comprising or consisting of the sequence set forth in SEQ ID NO: 1.

### BRIEF DESCRIPTION OF THE DRAWINGS

Embodiments of the invention are described herein, by way of non-limiting example only, with reference to the following figures.

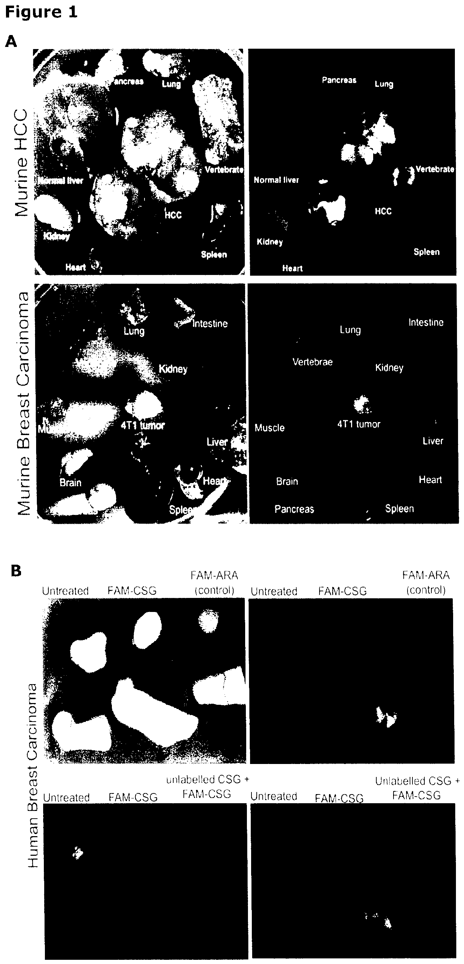

**Figure 1****.** CSG specifically accumulates in tumours. **A.** Macroscopic images of tissues indicating fluorescein-labelled (FAM)-CSG accumulates in hepatocellular carcinoma (upper) and breast carcinoma (lower). Homing was specific to tumours, with only clearance organs (intestine and kidney) showing some binding. **B.** Upper: fresh, untreated samples of human breast carcinoma dipped in FAM-CSG. Peptide homing was observed in tumours (T= tumours), not in normal (N) or marginal (M) tissues. Control peptide FAM-ARA showed no binding. Lower: pre-incubation with an excess of unlabelled CSG peptide abolished the FAM-CSG specific penetration and accumulation in tumours.

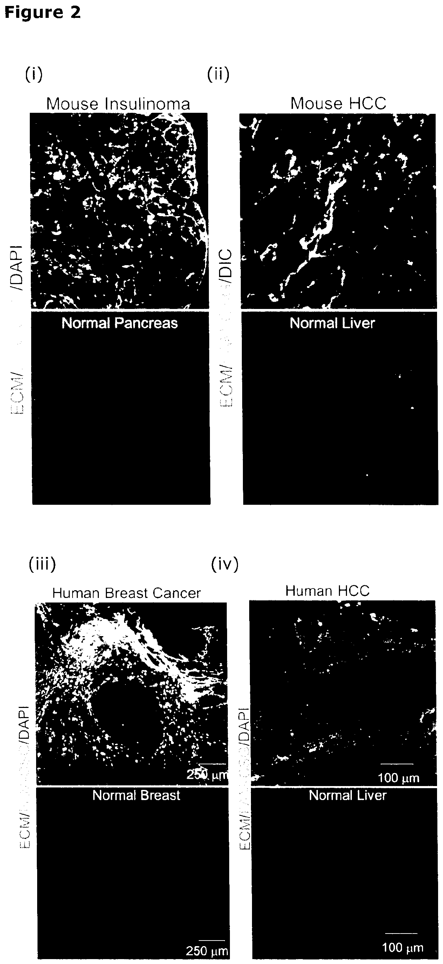

**Figure 2****.** CSG accumulation and binding in tumours colocalises exclusively with tumour ECM. Co-staining of CSG with (i) nidogen-1 in mouse insulinoma compared to normal pancreas, (ii) nidogen-1 in mouse hepatocellular carcinoma compared to normal liver, (iii) collagen-1 in human breast carcinoma compared to normal breast tissue and (iv) collagen-1 in human HCC compared to non-tumour fatty liver. In each case, the images clearly show colocalisation of CSG with ECM markers.

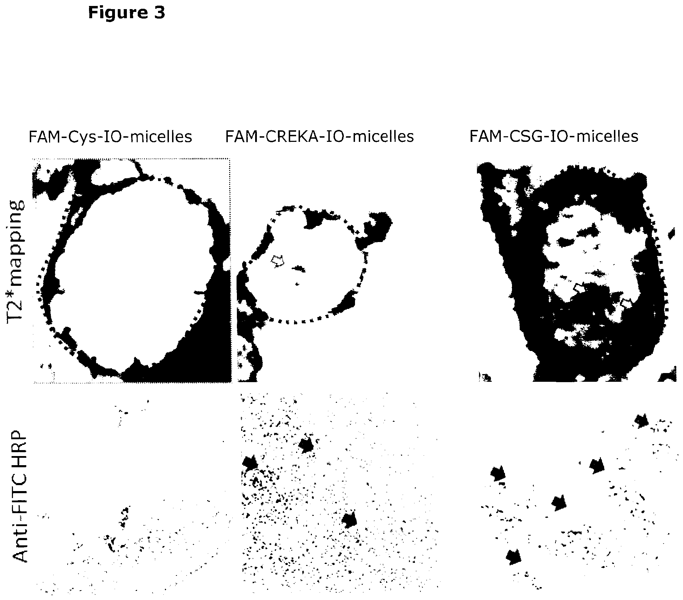

**Figure 3****.** CSG targeting of tumour ECM enhances the delivery of imaging compounds, more effectively than CREKA targeting of tumour blood vessels. Insulinoma bearing mice were intravenously injected with 100 µL of 1 mM fluorescein-labelled (FAM) untargeted iron oxide (IO) micelles (FAM-Cys-IO-micelles), CREKA-tagged IO micelles (FAM-CREKA-IO-micelles) or CSG-tagged IO micelles (FAM-CSG-IO-micelles). Tumours were harvested after heart perfusion and imaged *ex vivo* by MRI and microscopic analysis. The MRI scan shows increased in accumulation of CSG-tagged IO micelles in injected tumours shown by T2* mapping (top) and histological detection of antibody against FAM (anti-FITC ab) (bottom).

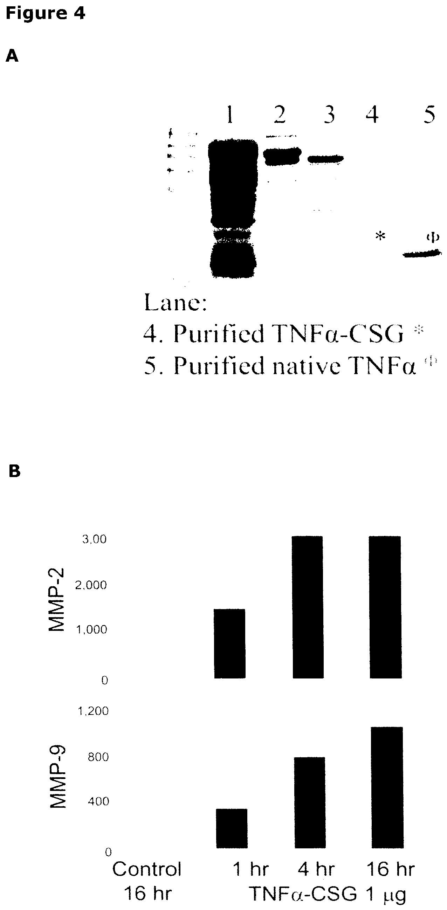

**Figure 4****.** Production of biologically active recombinant TNFα-CSG fusion protein specific for tumours. **A.** Fusion compound TNFα-CSG (TNFα-GGG-CSGRRSSKC; SEQ ID NO:4) purified using affinity chromatography based on Ni-NTA separation. **B.** TNFα-CSG bound to matrigel containing tumour ECM was able to stimulate macrophage cell line J744 to secrete protease degrading enzymes MMP-2 and MMP-9. **C.** Fluorescein-labelled TNFα-CSG and TNFα native protein accumulation in tumours and normal tissues evaluated by histology using anti-fluorescein (anti-FITC) antibody. TNFα-CSG homed to tumours and has limited binding in normal tissues such as pancreas, liver, heart and kidney.

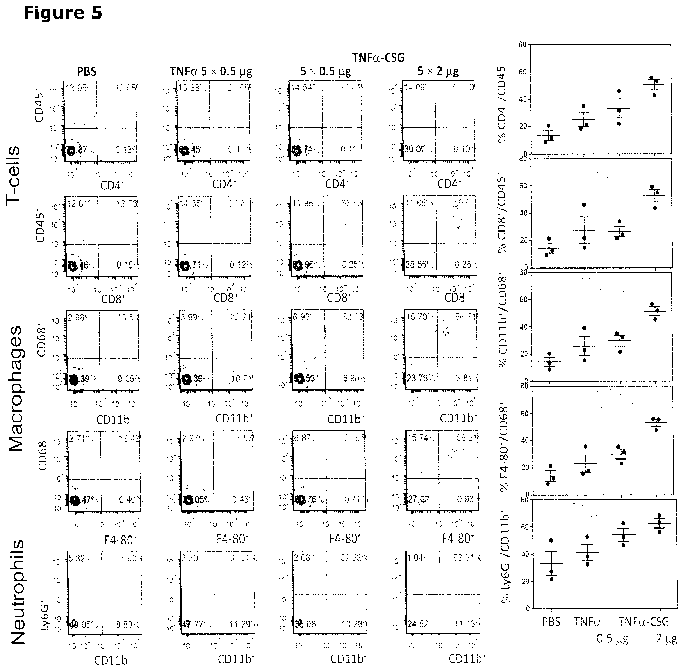

**Figure 5****.** TNFα-CSG treatment enhances immune cell infiltration in 4T1 breast carcinomas. FACS quantification of CD4⁺ and CD8⁺ T cells and infiltrating macrophages (CDllb⁺/ CD68⁺/ F4/80⁺) harvested in whole tumours. Quantitative analysis of tumours shows an increase in immune cell infiltration is most effective in tumours treated with 2 µg TNFα-CSG (*P*< *0.02).*

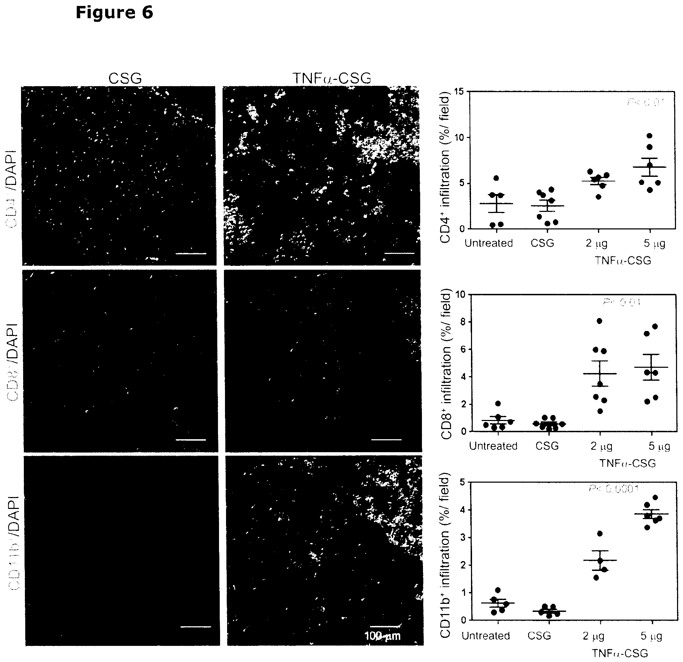

**Figure 6****.** TNFα-CSG treatment enhances immune cell infiltration in RIP-Tag insulinomas. Mice bearing RIP-Tag insulinoma at 25 weeks of age were treated with 2 and 5 µg TNFα-CSG daily intravenous injection for 5 days. Microscopic evaluation of immune cells CD4+ and CD8+ T cells and circulatory CD11b+ macrophages detected by immunofluorescence staining of tissue cross sections. Quantitative analysis shows significant increase in immune cell infiltration in TNFα-CSG treated tumours (P< 0.01) at both 2 and 5 µg TNFα-CSG.

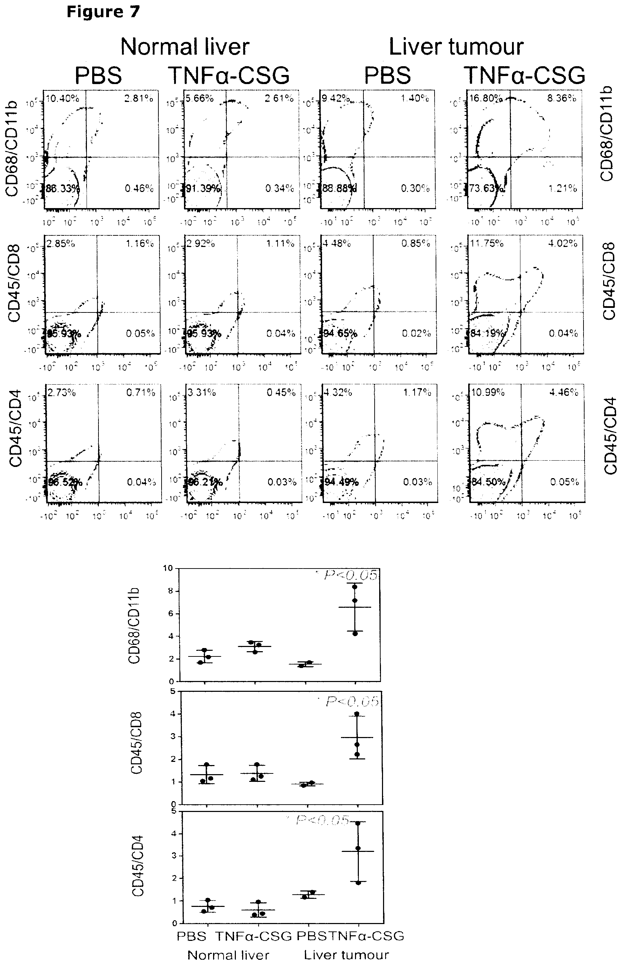

**Figure 7****.** TNFα-CSG treatment enhances immune cell infiltration in ALB-Tag hepatocellular carcinomas. FACS quantification of CD4⁺ and CD8⁺ T cells and infiltrating macrophages (CD11b⁺/CD68⁺) in whole tumours detected by immunofluorescence staining of tissue cross sections. Quantitative analysis shows at least a 3 fold increase in immune cell infiltration in HCC compared to control tumours *(P*<*0.05).*

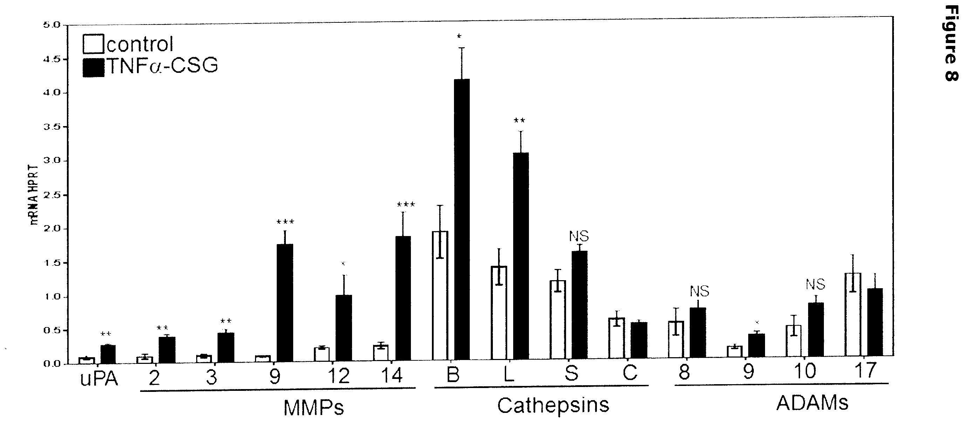

**Figure 8****.** Expression levels of proteases relative to the expression of hypoxanthine-guanine phosphoribosyl transferase (HPRT) in CD45+ leukocytes isolated from tumours following treatment with 0.8 µg CSG (control; grey (left hand) bars) or 10 µg TNFα-CSG (solid filled (right hand) bars) i.v. daily for 5 days. * P<0.05, ***P <0.001, ****P <0.0001 when compared to the CSG-treated control groups (n=5/group). Mean ± SD.

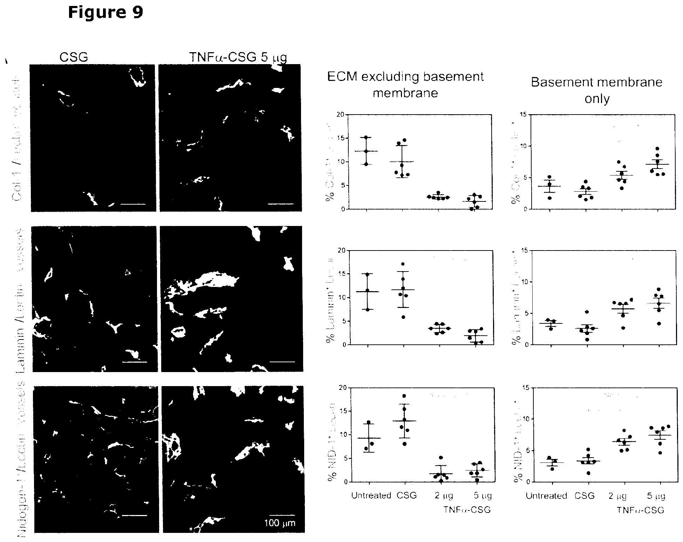

**Figure 9****.** TNFα-CSG treatment specifically reduces ECM content in tumours. Microscopic evaluation of ECM components collagen-1, laminin and nidogen-1 on tumour tissue cross sections showing TNF-CSG treated tumours with reduced in ECM contents. Quantitative analysis of ECM positive for collagen-1, laminin and nidogen 1, excluding basement membrane, indicate significant reduction in ECM content *(P*<*0.05)* in response to 2 and 5 µg TNFα-CSG.

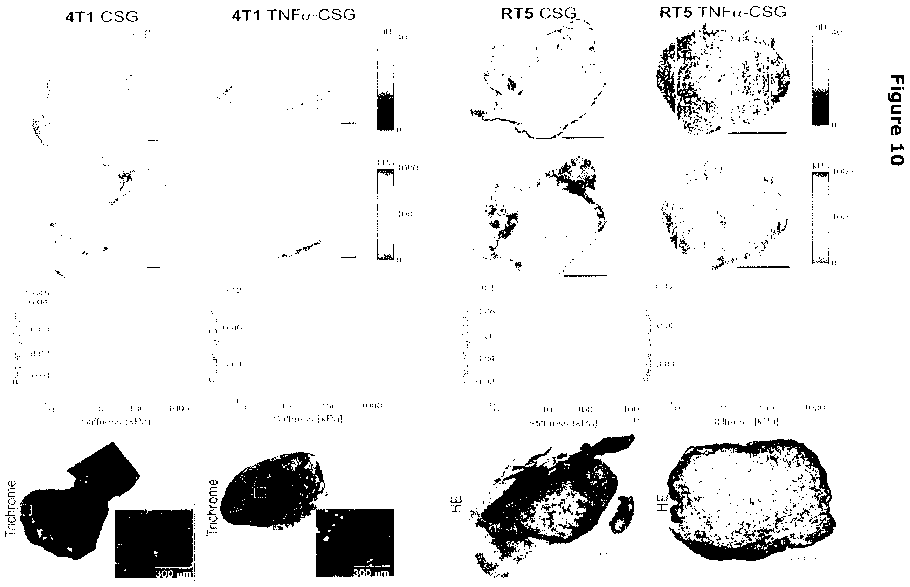

**Figure 10****.** TNFα-CSG treatment specifically reduces ECM content in 4T1 breast carcinoma and RIP-Tag insulinoma. Mapping of tumour stiffness by optical coherence tomography (OCT)/ micro-elastography on TNFα-CSG treated 4T1 tumour (left) and RIP-Tag insulinoma (right). En face OCT image at a depth of ∼500 µm (top) and corresponding (i) en face micro-elastogram (second), (ii) plot of stiffness distribution (third) and (iii) trichrome staining (bottom).

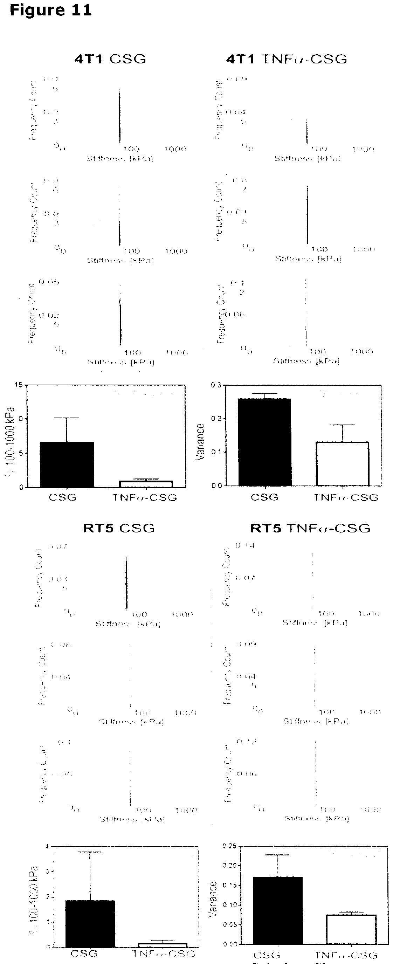

**Figure 11****.** Frequency distribution of tumour stiffness and stiffness variance based on OCT -elastography.

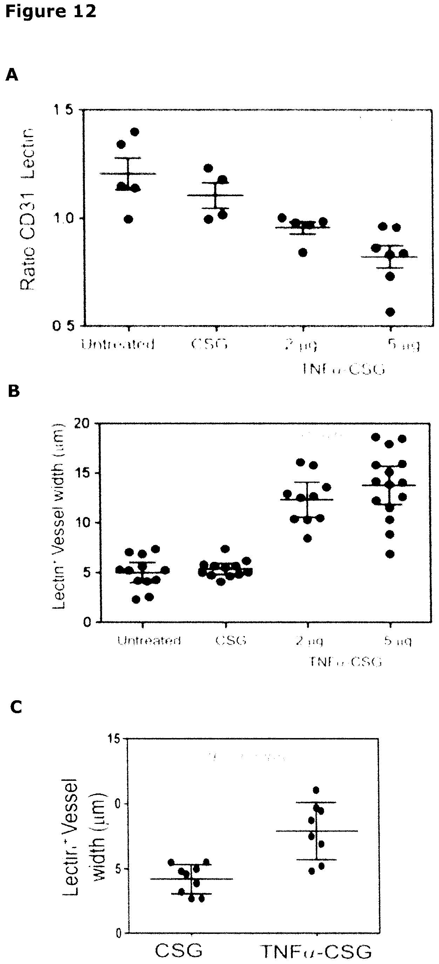

**Figure 12****.** Reduction in tumour stiffness significantly enhances blood perfusion in RIP-Tag insulinoma and 4T1 breast carcinoma. **A.** TNFα-CSG treatment significantly improved the overall blood perfusion in tumours, as evidenced by CD31:lectin ratio, compared to CSG treatment. **B. & C.** Vessels in TNFα-CSG treated tumours are significantly wider than in control treated tumours, and hence improved in perfusion compared to CSG-treated tumours.

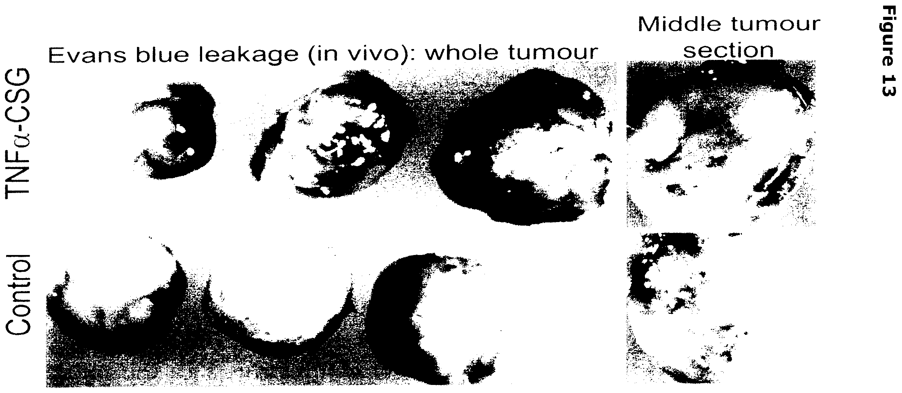

**Figure 13****.** Reduced tumour ECM and stiffness and improved perfusion results in more permeable tumours that are more susceptible to circulatory uptake of Evans Blue dye.

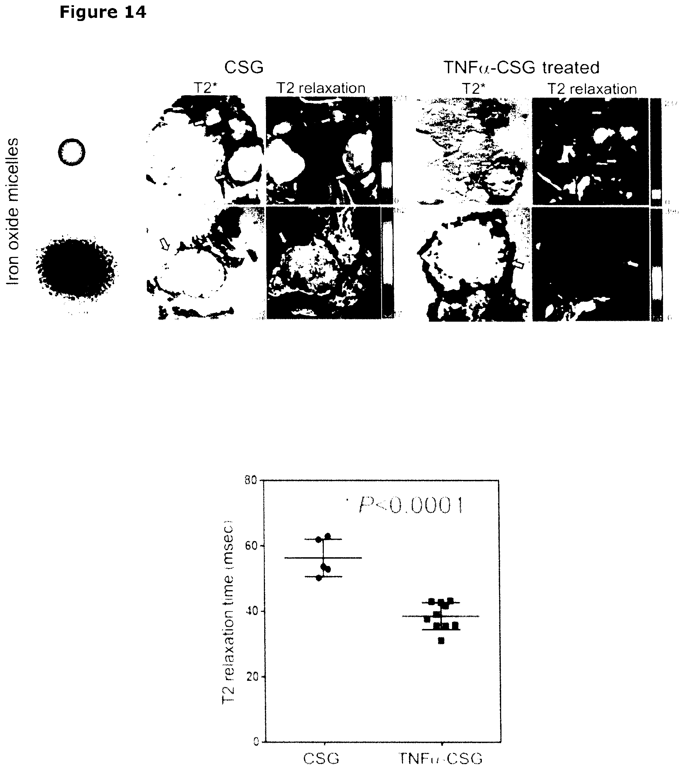

**Figure 14****.** Reduced tumour ECM and stiffness and improved perfusion results in enhanced tumour uptake of a nano-imaging contrast agent. **A.** Insulinoma bearing mice were intravenously injected with 100 µL of 1 mM iron oxide (IO) micelles. Tumours were harvested and imaged by MRI and microscopic analysis. The MRI scan shows increased in accumulation of FITC-labelled IO micelles in TNFα-CSG treated tumours (2 µg dose) shown by T2* (dark contrast) and T2 relaxation. The loss of signal (i.e. T2 relaxation time msec) is significantly lower (representing greater iron oxide micelle accumulation).

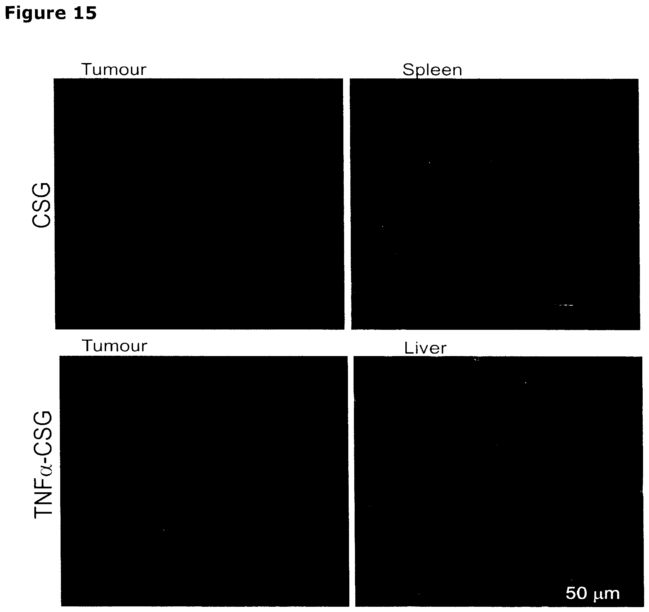

**Figure 15****.** Improved doxo-micelles accumulate in TNFα-CSG treated tumours. 4T1 tumour-bearing mice were treated with 2 µg TNFα-CSG or CSG control by intravenous injection. Mice were then injected with 100 µL of 1 mM doxorubicin-micelles. Microscopic evaluation shows strong traces of doxorubicin in TNFα-CSG treated tumours, comparable to the non-specific uptake of doxorubicin micelles in spleen and liver.

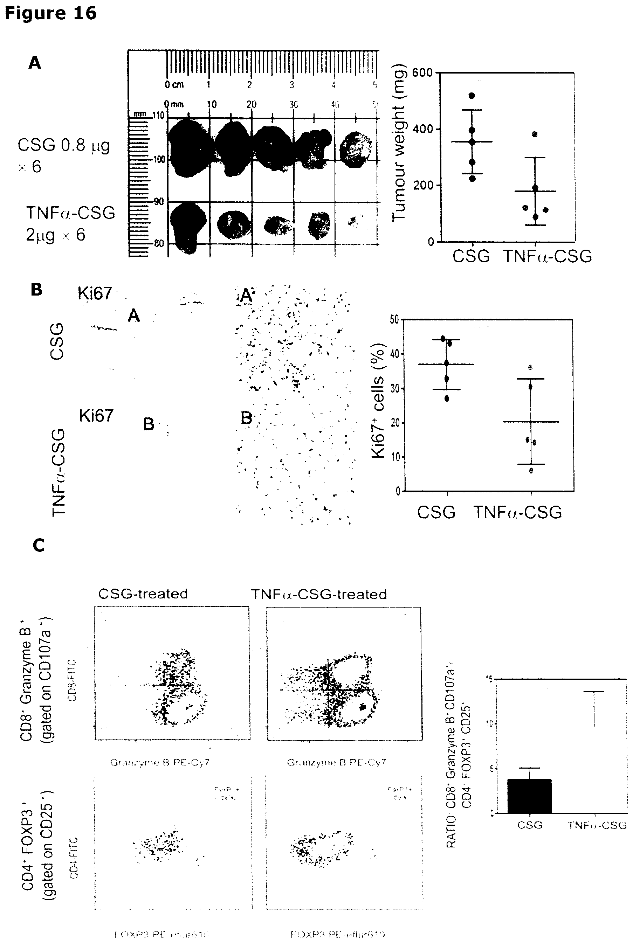

**Figure 16****.** TNFα-CSG has anti-tumorigenic effects. **A.** Comparison of tumour size and weight indicating significantly reduced tumour growth in response to TNFα-CSG treatment. **B.** Microscopic evaluation of whole tumours for cell proliferation marker (Ki67+ staining) indicating significant reduction in tumour cell proliferation in TNFα-CSG treated tumours. C. FACs quantification analysis of infiltrating T cell populations that indicates higher levels of CD8+ cytotoxic T cells expressing granzyme B and CD107a markers, and lower levels of CD4+ T cells expressing FoxP3 and CD25 markers. Bar graph indicates significantly higher ratio of CD8+ cytotoxic T cells compare to CD4+ regulatory T cells in the TNF α-CSG treated tumours compare to control.

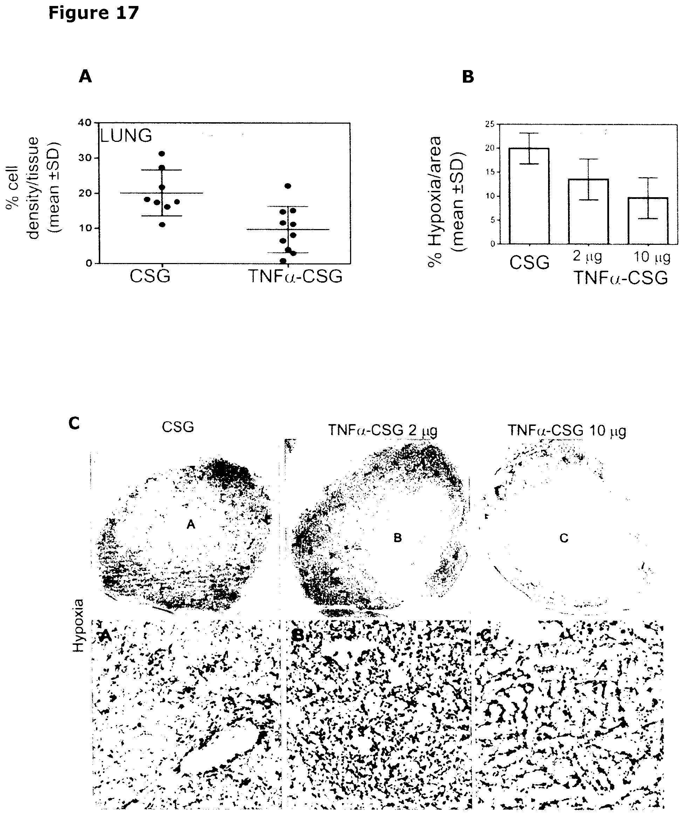

**Figure 17****.** TNFα-CSG reduces secondary metastasis of tumours. **A.** 4T1 tumour-bearing mice were treated with TNFα-CSG or control CSG peptide daily once primary tumours reached at least 500 mm³. Quantitative analysis of cell density/tissue (% mean ± SD) indicates significantly reduced lung metastasis in TNFα-CSG treated group. **B.** Reduced metastasis correlated with significantly reduced hypoxia in primary tumours. **C.** Treatment with TNFα-CSG showed an enlarged tumour centre cleared of tumour cells.

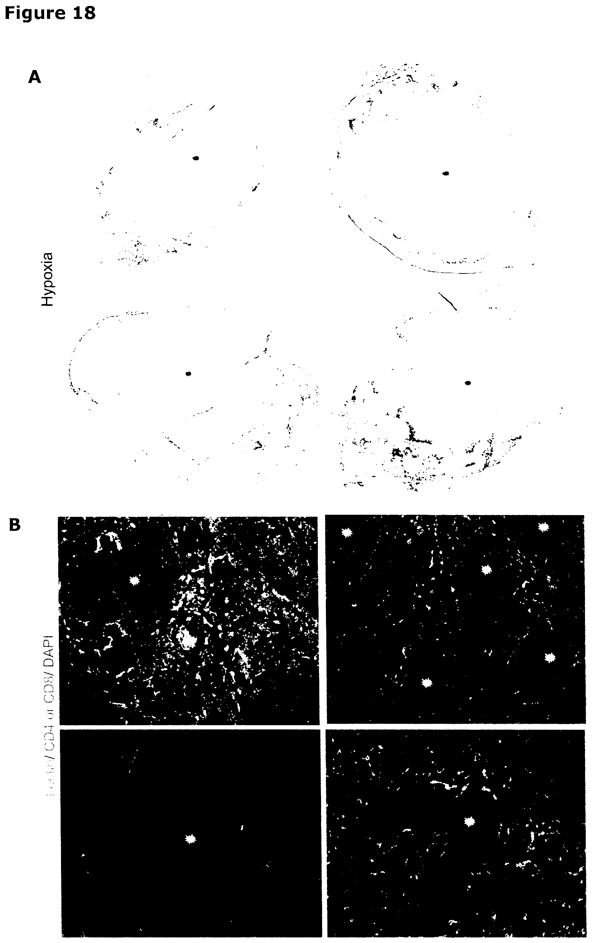

**Figure 18****.** Evidence of tumour clearance in response to TNFα-CSG treatment. **A.** Whole microscopic images of 4T1 tumours following TNFα-CSG treatment. The tumours were stained with hypoxia marker. The middle region of all tumours showed areas cleared of tumour cells. **B.** Microscopic images of RIP-Tag tumours following TNFα-CSG treatment. The tumours were stained with immune cell markers (CD4 and CD8) as well as lectin. The middle areas of many tumours were devoid of tumour cells.

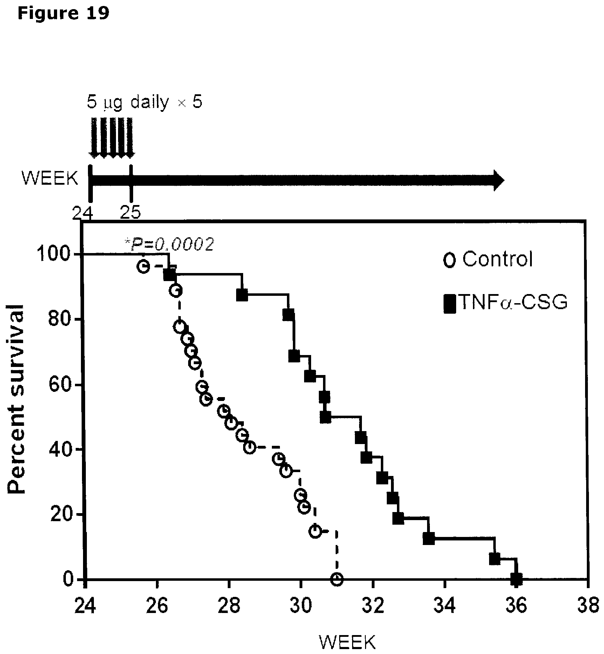

**Figure 19****.** Long-term survival under TNFα-CSG monotherapy (***P<0.0005). Mice bearing advanced insulinoma at 24 weeks of age were treated with TNFα-CSG (5 µg i.v. injection daily for 5 days). Animals were monitored for survival up to 38 weeks.

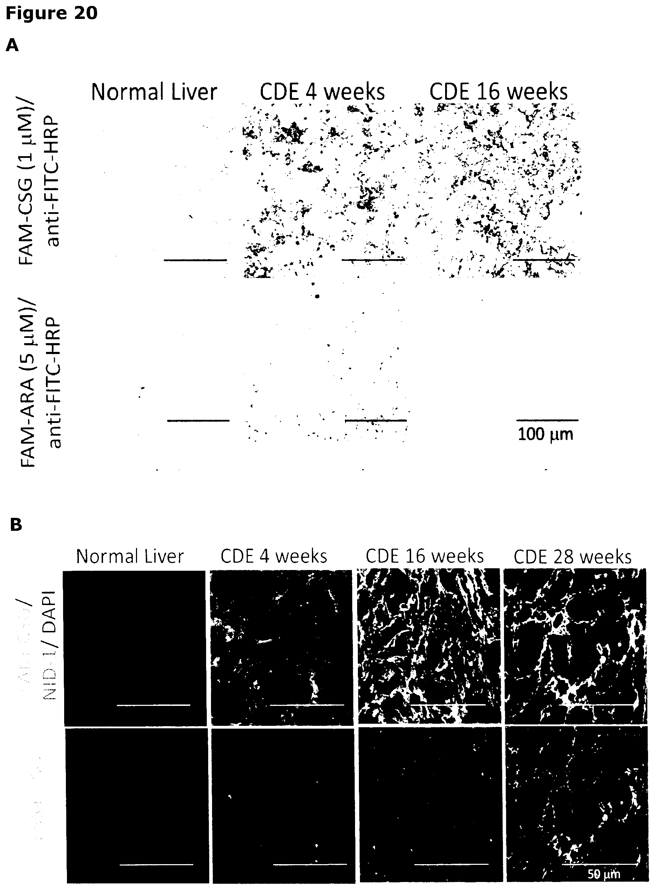

**Figure 20****.** CSG targets fibrotic tissue. **A.** FAM-CSG specifically binds to early liver fibrotic tissue (4 weeks after fed with choline deficient diet, CDE), advanced fibrosis-cirrhosis (16 weeks CDE) and advanced HCC (28 weeks CDE). Normal liver from mice fed with normal chow diet shows limited binding. **B.** FAM-CSG binding co-localises with nidogen-1 staining.

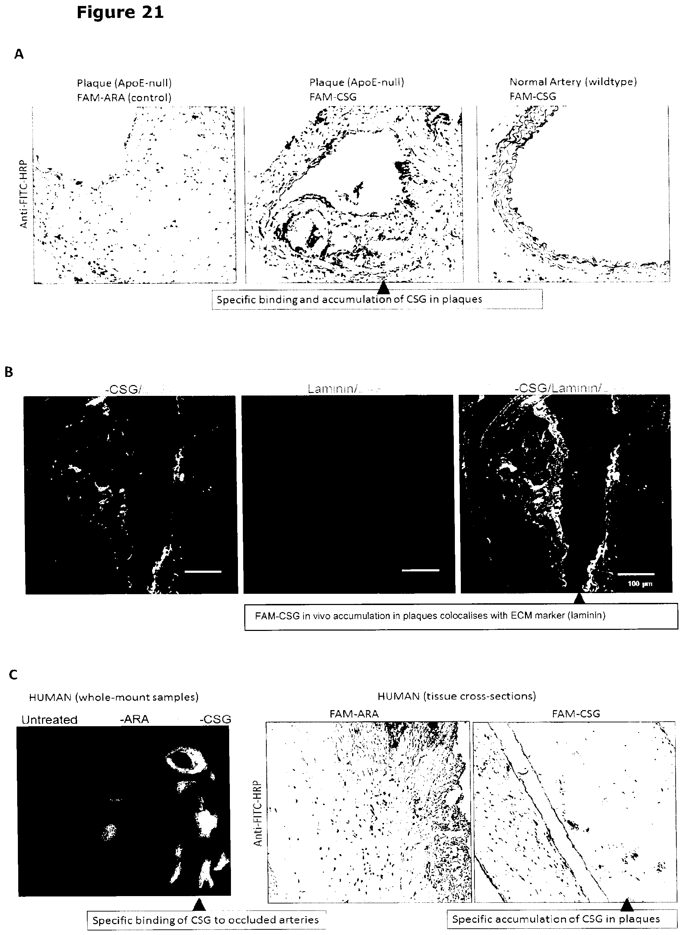

**Figure 21****.** Healthy aorta and aorta containing plaques from wildtype C57BL/6 and ApoE-null mice, respectively, were incubated with 20 nmol/mL fluorescein-labelled FAM-CSG or ARA control. **A.** Tissue cross sections were stained with anti-fluorescein-HRP antibody, highlighting areas within the tissue positive for peptide accumulation. **B.** Immunostaining of tissue cross section of aorta containing plaque following 1 hr intravenous injection of 100 µL 1 mM FAM-CSG, showing co-localisation of FAM-CSG and laminin (right hand image). **C.** Occluded artery sample from a human patient with occlusive peripheral vascular disease after limb amputation subjected to a dipping assay with FAM-labeled peptides (left). The corresponding tissue cross sections (right) stained with anti-FITC-HRP, show areas within plaque positive for CSG binding.

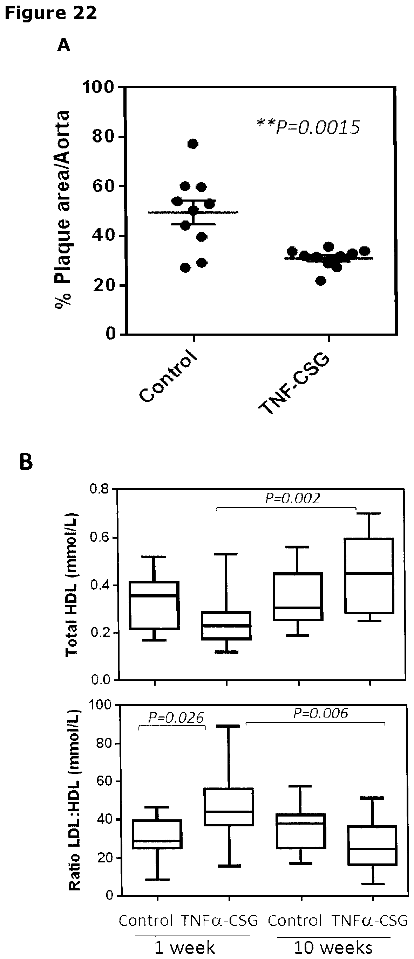

**Figure 22****.** Aging ApoE null mice (at 59 and 69 weeks of age, n=10-13/group male only) were treated with TNFα-CSG (daily i.v. dose of 10 µg TNFα-CSG, control untreated or CSG peptide × 5 days). Tissues including aorta and plasma were collected from euthanised animals at 70 weeks of age. Plasma samples were collected after 1 and 10 weeks of therapy. **A.** Quantification of plaque positive area (% mean ± SE) indicates TNFα-CSG therapy significantly reduced plaque burden. **B.** Box and Whisker plots of plasma HDL cholesterol and ratio LDL: HDL cholesterol, indicating the plasma LDL:HDL ratio increased after 1 week of TNFα-CSG therapy. The plasma HDL cholesterol was significantly increased and LDL:HDL ratio was reduced 10 weeks after TNFα-CSG therapy.

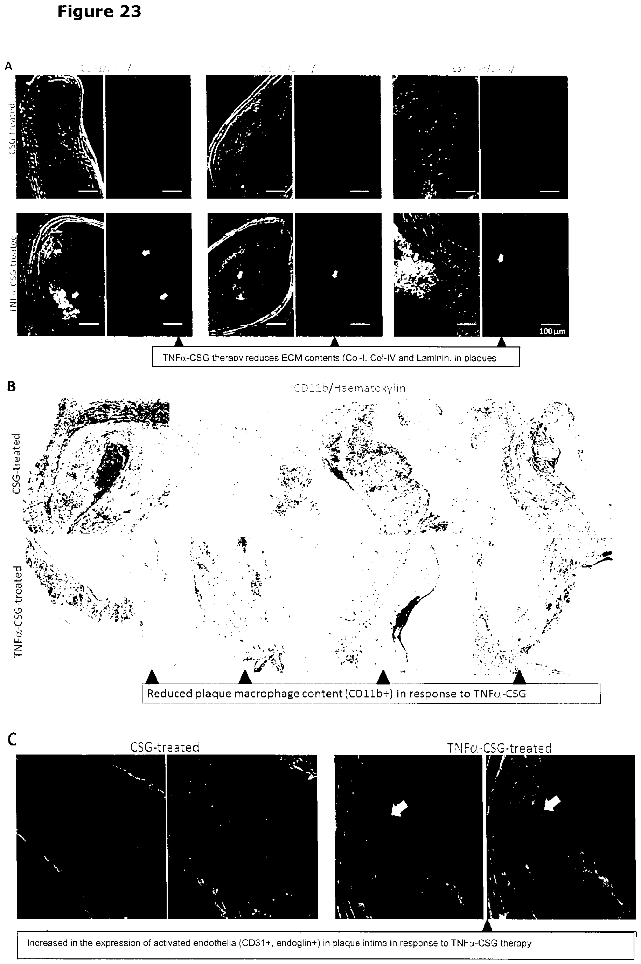

**Figure 23****.** Comparison of representative tissue cross-sections of aorta containing plaques after 1 week of TNFα-CSG treatment showing: **A.** reduced immune-staining of plaque expression of collagen-I, Collagen-IV and laminin (the lack of ECM contents is indicated by arrow). **B.** reduced macrophage (immunoperoxidase staining of CD11b macrophage marker), and **C.** upregulated endothelia positive for CD31 and endoglin markers (shown by arrow) within the plaque interior.

A listing of amino acid sequences corresponding to the sequence identifiers referred to in the specification is provided in a formal sequence listing appearing at the end of the specification.

### DETAILED DESCRIPTION

The articles "a" and "an" are used herein to refer to one or to more than one (*i.e.,* to at least one) of the grammatical object of the article. By way of example, "an element" means one element or more than one element.

Throughout this specification and the claims which follow, unless the context requires otherwise, the word "comprise", and variations such as "comprises" or "comprising", will be understood to imply the inclusion of a stated integer or step or group of integers or steps but not the exclusion of any other integer or step or group of integers or steps.

In the context of this specification, the term "about," is understood to refer to a range of numbers that a person of skill in the art would consider equivalent to the recited value in the context of achieving the same function or result.

The term "peptide" means a polymer made up of amino acids linked together by peptide bonds. The term "polypeptide" may also be used to refer to such a polymer although in some instances a polypeptide may be longer (i.e. composed of more amino acid residues) than a peptide. Notwithstanding, the terms "peptide" and "polypeptide" may be used interchangeably herein.

As used herein the terms "treating", "treatment", "preventing", "prevention" and grammatical equivalents refer to any and all uses which remedy a disease or condition, prevent, retard or delay the establishment of a disease or condition, or otherwise prevent, hinder, retard, or reverse the progression of a disease or condition. Thus the terms "treating" and "preventing" and the like are to be considered in their broadest context. For example, treatment does not necessarily imply that a patient is treated until total recovery. In diseases and conditions which display or a characterized by multiple symptoms, the treatment or prevention need not necessarily remedy, prevent, hinder, retard, or reverse all of said symptoms, but may prevent, hinder, retard, or reverse one or more of said symptoms.

As used herein the term "effective amount" includes within its meaning a non-toxic but sufficient amount or dose of an agent or compound to provide the desired effect. The exact amount or dose required will vary from subject to subject depending on factors such as the species being treated, the age, size, weight and general condition of the subject, the severity of the disease or condition being treated, the particular agent being administered and the mode of administration and so forth. Thus, it is not possible to specify an exact "effective amount". However, for any given case, an appropriate "effective amount" may be determined by one of ordinary skill in the art using only routine experimentation.

As used herein the term "sensitivity" is used in its broadest context to refer to the ability of a cell to survive exposure to an agent designed to inhibit the growth of the cell, kill the cell or inhibit one or more cellular functions.

As used herein the term "resistance" is used in its broadest context to refer to the reduced effectiveness of a therapeutic agent to inhibit the growth of a cell, kill a cell or inhibit one or more cellular functions, and to the ability of a cell to survive exposure to an agent designed to inhibit the growth of the cell, kill the cell or inhibit one or more cellular functions. The resistance displayed by a cell may be acquired, for example by prior exposure to the agent, or may be inherent or innate. The resistance displayed by a cell may be complete in that the agent is rendered completely ineffective against the cell, or may be partial in that the effectiveness of the agent is reduced.

The term "subject" as used herein refers to mammals and includes humans, primates, livestock animals (e.g. sheep, pigs, cattle, horses, donkeys), laboratory test animals (e.g. mice, rabbits, rats, guinea pigs), performance and show animals (e.g. horses, livestock, dogs, cats), companion animals (e.g. dogs, cats) and captive wild animals. Preferably, the mammal is human or a laboratory test animal. Even more preferably, the mammal is a human.

As used herein, the term "immunopotentiating cytokine" refers to a cytokine that can mediate a cellular immune response. Cytokines suitable for use in the invention include, but are not limited to TNFα, interferons (IFNs) such as IFNy, IFN-1β, IFNα, interleukins (ILs) such as IL-1, IL-2, IL-1β, IL-6, IL-7, IL-8, IL-12, IL-15, IL-18, IL-21, and granulocyte-macrophage colony-stimulating factor (GM-CSF). An immunopotentiating cytokine may mediate the cellular immune response by stimulating the expression and/or secretion of proteases, and/or the chemoattraction of immune cells. Proteases mediated by immunopotentiating cytokines may include, but are not limited to, matrix metalloproteases, disintegrins, cathepsins and metalloprotease (ADAM) family members such as ADAM-10 and ADAM-17, elastase and collagenase.

The present inventors have generated novel biomolecule conjugates comprising TNFα or IFNγ and a peptide comprising or consisting of the sequence set forth in SEQ ID NO: 1. However the person skilled in the art will recognise that the invention is not limited to use of TNFα or IFNγ as the conjugating cytokine, and that TNFα or IFNγ may be substituted with any cytokine, optionally an immunopotentiating cytokine.

Conjugates according to the present invention are referred to hereinbelow as "TNFα-CSG" and "IFNy-CSG". This descriptor is used for simplicity and convenience only, and should not be taken as in any way limiting the scope of the invention; the reference to "CSG" means a peptide (or polypeptide) comprising or consisting of the sequence set forth in SEQ ID NO:1, which sequence includes the triplet "CSG".

As exemplified herein the inventors have demonstrated that TNFα-CSG and IFNγ-CSG home to tumour ECM *in vivo.* Systemic administration of TNFα-CSG and IFNγ-CSG in mice bearing breast carcinoma and insulinoma is shown to trigger significant immune cell infiltration that engage exclusively with tumour ECM. It is also shown that TNFα-CSG treatment induces an increase in the expression and/or secretion of a large number of proteases locally, significantly degrades tumour ECM content and reduces tumour stiffness and blood vessel compression, thus enabling significantly increased perfusion. Consequently, TNFα-CSG treated tumours are interstitially more accessible to circulatory uptake of circulating reagents, including imaging and therapeutic agents. The effect of TNFα-CSG on tumours can thus be exploited to improve cancer detection and imaging, as an anti-cancer monotherapy and as an adjunct to existing therapies such as chemotherapy, immunotherapy and radiotherapy whilst minimising the risk of tumour metastasis. Without wishing to be bound by theory, the inventors suggest that the local increase in expression and/or secretion of multiple proteases by the TNFα-CSG is critical in the significant levels of ECM degradation observed, and that this surprising finding offers a distinct advantage over current therapies not only in terms of the amount of ECM degradation due to the inducement of multiple proteases, but also due to the fact that protease release is local to the affected site thereby avoiding the toxicity associated with systemic protease production and activity.

Without wishing to be bound by theory, the inventors suggest that the TNFα-CSG induces or promotes infiltration of tumours by immune cells that release ECM-degrading proteases. The degradation of tumour ECM, and consequent reduction of tumour stiffness, induced by TNFα-CSG results in reduced compression, in turn leading to an expansion in tumour vessels and increased perfusion.

The present disclosure also exemplifies the ability of CSG to localise to atherosclerotic plaques, and to specifically target and bind atherosclerotic plaque ECM. Further, the inventors have shown that TNFα-CSG reduces atherosclerotic plaque formation, degrades ECM in plaque intima, reduces macrophage content and increases the expression of activated endothelia in plaque intima.

Also exemplified herein is the ability of CSG to localise to fibrotic tissue, and specifically target and bind the abnormal ECM associated with fibrotic tissue from the earliest sign of fibrosis development. Accordingly, the inventors postulate that the inducement or promotion of infiltration by immune cells and the ECM degradation observed in tumours and atherosclerotic plaques in the presence of TNFα-CSG also applies to fibrotic tissue.

An aspect of the invention provides a biomolecule conjugate comprising a cytokine, optionally an immunopotentiating cytokine, and a peptide comprising or consisting of the sequence set forth in SEQ ID NO:1. In exemplary embodiments, the immunopotentiating cytokine is TNFα or IFNγ.

Also provided is a biomolecule conjugate for use in methods for degrading the extracellular matrix (ECM) of tumour, atherosclerotic or fibrotic tissue, comprising exposing the tumour, atherosclerotic or fibrotic tissue to an effective amount of the biomolecule conjugate.

Also provided is a biomolecule conjugate for use in methods for promoting or inducing immune cell infiltration of a tumour or of atherosclerotic or fibrotic tissue, comprising exposing the tumour or atherosclerotic or fibrotic tissue to an effective amount of the biomolecule conjugate.

Also provided is a biomolecule conjugate for use in methods for treating conditions associated with abnormal ECM, optionally selected from tumours, atherosclerosis and fibrosis, comprising administering to a subject in need thereof an effective amount of the biomolecule conjugate.

Also provided is a biomolecule conjugate for use in methods for treating solid tumours, atherosclerosis and fibrosis, comprising administering to a subject in need thereof an effective amount of the biomolecule conjugate. The invention also provides a biomolecule conjugate for use in methods for increasing or extending the survival of subjects having a tumour, comprising administering to a subject in need thereof an effective amount of the biomolecule conjugate.

The 'CSG' peptide for use in accordance with aspects and embodiments of the present invention comprises or consists of the peptide sequence CSGRRSSKC (SEQ ID NO:1), or conservative variants thereof. Conservative variants comprise one or more conservative amino acid substitutions, being the substitution or replacement of one amino acid for another amino acid with similar properties as would be well understood by those skilled in the art. For example, the substitution of the neutral amino acid serine (S) for the similarly neutral amino acid threonine (T) would be a conservative amino acid substitution. Those skilled in the art will be able to determine suitable conservative amino acid substitutions that do not eliminate the ability of the peptide to specifically target tumour ECM.

A peptide sequence comprising the sequence CSGRRSSKC (SEQ ID NO:1) may have, for example, a relatively short length often, 11, 12, 13, 14, 15, 16, 17, 18, 19, 20, 25, 30, 35, 40, 45, 50, 60, 70 or 80 residues, typically as a contiguous sequence. Alternatively, the peptide may retain the tumour and fibrosis ECM homing activity of 'CSG' when provided in the context of (e.g. embedded in) a larger peptide, polypeptide or protein sequence. Thus, the invention further provides chimeric peptides, polypeptides and proteins containing the sequence CSGRRSSKC (SEQ ID NO:1) fused to a heterologous peptide, polypeptide or protein. Such a chimeric peptide, polypeptide or protein may have a length of, for example, up to about 10, 15, 20, 25, 30, 35, 40, 45, 50, 55, 60, 65, 70, 75, 80, 85, 90, 95, 100, 150, 200, 250, 300, 350, 400, 450, 500, 800, 1000 or 2000 residues or more.

Peptidomimetics of the 'CSG' peptide are also contemplated and encompassed by the present disclosure. The term "peptidomimetic," as used herein means a peptide-like molecule that has the tumour and fibrosis homing activity of the peptide upon which it is structurally based. Such peptidomimetics include chemically modified peptides, peptide-like molecules containing non-naturally occurring amino acids, and peptoids (see, for example, Goodman and Ro, Peptidomimetics for Drug Design, in "Burger's Medicinal Chemistry and Drug Discovery" Vol. 1 (ed. M. E. Wolff; John Wiley & Sons 1995), pages 803-861).

A variety of peptidomimetics are known in the art including, for example, peptide-like molecules which contain a constrained amino acid (for example an α-methylated amino acid, α,α-dialkyl glycine, α-, β- or γ-aminocycloalkane carboxylic acid, an α,β-unsatruated amino acid, a β,β-dimethyl or β-methyl amino acid or other amino acid mimetic), a non-peptide component that mimics peptide secondary structure (for example a nonpeptidic 3-turn mimic, γ-turn mimic, a mimic of β sheet structure, or a mimic of helical structure), or an amide bond isostere (for example a reduced amide bond, methylene ether bond, ethylene bond, thioamide bond or other amide isostere). Methods for identifying peptidomimetics are also well known in the art and include, for example, the screening of databases that contain libraries of potential peptidomimetics.

The peptide or peptidomimetic may be cyclic or otherwise conformationally constrained. Conformationally constrained molecules can have improved properties such as increased affinity, metabolic stability, membrane permeability or solubility. Methods of conformational constraint are well known in the art.

A cytokine for use in accordance with the invention may be a human cytokine. The cytokine may comprise the native human sequence of the mature cytokine or a derivative, variant or homologue thereof. Precursor, recombinant or modified forms of the cytokine may also be used. In the context of the present specification reference to variants, homologues and modified forms of cytokines have the same meaning as are given below in relation to the exemplary cytokine TNFα.

The TNFα may comprise the native mature human TNFα sequence. Precursor and recombinant forms of TNFα may also be employed. TNFα is predominantly synthesised by immune cells such as T cells and activated monocytes and macrophages as a pro-protein which localises to the plasma membrane. Proteolytic cleavage of the cell-bound pro-TNFa by metalloproteases such as TNFα-converting enzyme (TACE) or TACE/a disintegrin-like metalloprotease (ADAM)-17 results in mature, soluble TNFα. The amino acid sequence of human pro-TNFα is provided in UniProt Accession No. P01375 (set forth herein in SEQ ID NO:2). Cleavage of pro-TNFa occurs at a cleavage site between amino acids 76 and 77 to generate mature TNFα. The amino acid sequence of mature (cleaved or processed) human TNFα is provided in UniProt Accession No. PRO_0000034424 (set forth herein in SEQ ID NO:3).

The IFNγ may comprise the native mature human IFNγ sequence. Precursor and recombinant forms of IFNγ may also be employed. The amino acid sequence of human precursor IFNγ is provided in UniProt Accession No. P01579 (set forth herein in SEQ ID NO:7). Cleavage of this precursor occurs at a cleavage site between amino acids 23 and 24 to generate mature IFNγ. The amino acid sequence of mature (cleaved or processed) human IFNγ is provided in NCBI Reference Sequence NP_000610 (set forth herein in SEQ ID NO:8).

The disclosure hereinbelow is made with reference to TNFα, however the skilled addressee will appreciate that the disclosure also applies to other cytokines, optionally immunopotentiating cytokines, contemplated herein, and it should therefore be understood that the reference to TNFα is exemplary and for convenience only. The scope of the following disclosure is not intended to be limited thereto.

Embodiments also contemplate the employment of variants and other modified forms of TNFα. Those skilled in the art will appreciate that the scope of the present invention is not limited by any specific sequence of TNFα used in constructing the biomolecule conjugate.

The term "variant" as used herein refers to substantially similar sequences that possess qualitative biological activity in common. These sequence variants may share at least 50%, 55%, 60%, 65%, 70%, 75%, 80%, 85%, 90%, 91%, 92%, 93%, 94%, 95%, 96%, 97%, 98% or 99% sequence identity. The term "sequence identity or "percentage of sequence identity" may be determined by comparing two optimally aligned sequences or subsequences over a comparison window or span, wherein the portion of the polynucleotide sequence in the comparison window may optionally comprise additions or deletions (i.e., gaps) as compared to the reference sequence (which does not comprise additions or deletions) for optimal alignment of the two sequences.

Also included within the meaning of the term "variant" are homologues of human TNFα. A homologue is typically a polypeptide or protein from a different species but sharing substantially the same biological function or activity as the corresponding human TNFα. Further, the term "variant" also includes modified derivatives of native TNFα polypeptides, or homologues thereof, which modified derivatives may comprises addition, deletion, substitution of one or more amino acids, such that the polypeptide typically retains substantially the same function.

The 'CSG' peptide may be conjugated to the C-terminal or N-terminal end of TNFα or other cytokine. The component molecules can be conjugated using standard chemical coupling techniques such as MBS, glutaraldehyde, EDC, or BDB coupling, or may be linked by peptide synthesis methods or recombinant methods. Accordingly, provided herein are biomolecule conjugates representing fusion proteins.

A peptide linker or spacer may be used to link the component molecules. Peptide linkers typically are from 1 amino acid in length to 10 amino acids in length, although can be longer. In exemplary embodiments the linker comprises one or more, optionally two or more or three or more glycine (G) residues. Accordingly, in an exemplary embodiment the conjugate may comprise the sequence set forth in SEQ ID NO:4, representing the sequence CSGRRSSKC (SEQ ID NO:1) conjugated to the C-terminal end of native, mature human TNFα (SEQ ID NO:3) via a GGG linker. In another exemplary embodiment, the conjugate may comprise the sequence set forth in SEQ ID NO:9, representing the sequence CSGRRSSKC (SEQ ID NO:1) conjugated to the C-terminal end of native, mature human IFNγ (SEQ ID NO:8) via a GGG linker. Other suitable linkers will be known to persons skilled in the art, illustrative examples of which include peptides and polypeptides comprising N-terminal LPETG and N-terminal ACPP-alkyne, or a combination thereof.

The biomolecule conjugates and their component molecules as provided herein can be produced using any method known in the art, including chemical synthesis techniques, nucleic acid synthesis techniques, peptide synthesis techniques and/or recombinant techniques. In one example, a component polypeptide, such as a 'CSG' peptide is synthesized using the Fmoc-polyamide mode of solid-phase peptide synthesis. Other synthesis methods include solid phase t-Boc synthesis and liquid phase synthesis. Purification can be performed by any one, or a combination of, techniques such as re-crystallization, size exclusion chromatography, ionexchange chromatography, hydrophobic interaction chromatography and reverse-phase high performance liquid chromatography using, for example, acetonitril/water gradient separation.

In other examples, component polypeptides are produced using recombinant methods well known in the art. Nucleic acid encoding the polypeptides can be obtained by any suitable method, for example RT-PCR or synthesis of an oligonucleotide that encodes a polypeptide of the present invention. Accordingly, also provided herein are nucleic acid molecules encoding conjugates disclosed and contemplated herein. It is well within the skill of a skilled artisan to design a nucleic acid molecule that encodes component polypeptides, and biomolecule conjugates, as described herein.

Such nucleic acids can be cloned into a vector, such as, for example, an expression vector suitable for the expression system of choice, operably linked to regulatory sequences that facilitate expression of the heterologous nucleic acid molecule. Accordingly, also provided are vectors, including expression vectors, which contain a nucleic acid molecule encoding polypeptides and biomolecule conjugates described herein. Many expression vectors are available and known to those of skill in the art for the expression of polypeptides. The choice of expression vector is influenced by the choice of host expression system. Such selection is well within the level of skill of the skilled artisan. In general, expression vectors can include transcriptional promoters and optionally enhancers, translational signals, and transcriptional and translational termination signals. Expression vectors that are used for stable transformation typically have a selectable marker which allows selection and maintenance of the transformed cells. In some instances, the vector is a viral vector, such as an adeno-associated viral vector, lentiviral vector, or retroviral vector, which can be used to transduce cells *in vitro* or *in vivo.*

Biomolecule conjugates of the present invention may be linked to an affinity tag to, for example, facilitate purification. Exemplary affinity tags include, but are not limited to, Nus.Tag, His.Tag, chitin binding protein (CBP), maltose binding protein (MBP), glutathione-S-transferase (GST), FLAG, and HA tags. Detectable molecules, including, but not limited to, fluorescent or chemiluminescent molecules, or biotin or streptavidin, also can be linked.

The biomolecule conjugates of the present invention may include or be linked to one or more other moieties to facilitate transport, targeting, detection, purification, or another function. For example, biomolecule conjugates of the present invention may be linked to or contain a cell targeting moiety that facilitates targeting of the molecules to one or more particular cancer cell or tumour tissue types. The conjugates can be linked to the one or more other moieties by any method known in the art, including any chemical or recombinant method, where appropriate, resulting in the formation of covalent and/or non-covalent bonds between the molecule and the one or more other moieties.

Biomolecule conjugates of the present invention find application, *inter alia,* in therapeutic treatment and imaging of any cancerous tumour, including, but not limited to, those associated with: lung cancer, including small cell lung cancer and non-small cell lung cancer; pancreatic cancer, including insulinomas; bladder cancer; kidney cancer; breast cancer; brain cancer, including glioblastomas and medulloblastomas; neuroblastoma; head and neck cancer; thyroid cancer; breast carcinomas, including triple negative breast cancers; cervical cancer; prostate cancer; testicular cancer; ovarian cancer; endometrial cancer; rectal and colorectal cancer; stomach cancer; esophageal cancer; skin cancer, including melanomas and squamous cell carcinomas; oral cancer including squamous cell carcinoma; liver cancer, including human hepatocellular carcinona (HCC); lymphomas; sarcomas, including osteosarcomas, liposarcomas and fibrosarcomas.

Particular embodiments of the invention relating to therapeutic treatment of tumours contemplate combination treatments, wherein administration of the conjugate is in conjunction with one or more additional anti-tumour therapies. Such additional therapies may include, for example, radiotherapy, chemotherapy or immunotherapy. The nature and identity of suitable anti-tumour agents will depend, for example, on the nature or type of tumour to be treated. The identification and selection of suitable agents is well within the capabilities of the skilled addressee. The scope of the present disclosure is not limited to any one agent or type of agent and the following are provided by way of example only.

Suitable chemotherapeutic agents may be, for example, alkylating agents (such as cyclophosphamide, oxaliplatin, carboplatin, chloambucil, mechloethamine and melphalan), antimetabolites (such as methotrexate, fludarabione and folate antagonists) or alkaloids and other antitumour agents (such as vinca alkaloids, taxanes, camptothecin, doxorubicin, daunorubicin, idarubicin and mitoxantrone).

Immunotherapy may comprise, by way of example only, adoptive cell transfer or the administration of one or more anti-tumour or immune checkpoint inhibitors or tumour-specific vaccines. Adoptive cell transfer typically comprises the recovery of immune cells, typically T lymphocytes from a subject and introduction of these cells into a subject having a tumour to be treated. The cells for adoptive cell transfer may be derived from the tumour-bearing subject to be treated (autologous) or may be derived from a different subject (heterologous). Suitable immune checkpoint inhibitors include antibodies such as monoclonal antibodies, small molecules, peptides, oligonucleotides, mRNA therapeutics, bispecfic/trispecific/multispecific antibodies, domain antibodies, antibody fragments thereof, and other antibody-like molecules (such as nanobodies, affibodies, T and B cells, ImmTACs, Dual-Affinity Re-Targeting (DART) (antibody-like) bispecific therapeutic proteins, Anticalin (antibody-like) therapeutic proteins, Avimer (antibody-like) protein technology), against immune checkpoint pathways. Exemplary immune checkpoint antibodies include anti-CTLA4 antibodies (such as ipilimumab and tremelimumab), anti-PD-1 antibodies (such as MDX-1106 [also known as BMS-936558], MK3475, CT-011 and AMP-224), and antibodies against PDL1 (PD-1 ligand), LAG3 (lymphocyte activation gene 3), TIM3 (T cell membrane protein 3), B7-H3 and B7-H4 (see, for example, Pardoll, 2012). However these are provided by way of example only, and those skilled in the art will appreciate that other antibodies directed to T cells or antibodies directed to other tumour cell markers may be employed.

For such combination therapies, each component of the combination therapy may be administered at the same time, or sequentially in any order, or at different times, so as to provide the desired effect. Alternatively, the components may be formulated together in a single dosage unit as a combination product. When administered separately, it may be preferred for the components to be administered by the same route of administration, although it is not necessary for this to be so.

Particular embodiments disclosed herein contemplate the sensitization of tumours and tumour cells to chemotherapeutic agents, immunotherapy agents and radiotherapeutic agents using biomolecule conjugates as disclosed herein. The tumour or tumour cells may display resistance to the chemotherapeutic agent, immunotherapy agent or radiotherapeutic agent in the absence of treatment with the conjugate.

Embodiments of the present invention also therefore provide methods for determining a change in sensitivity of a tumour or tumour cell to a chemotherapeutic agent, immunotherapy agent or radiotherapeutic agent. Such methods may comprise
(a) administering to a subject a biomolecule conjugate according to the present invention;
(b) determining the sensitivity or resistance to the agent in a biological sample from the subject comprising at least one tumour cell;
(c) repeating steps (a) and (b) at least once over a period of time; and
(d) comparing said sensitivity or resistance in the samples.

Biomolecule conjugates of the present invention find application, *inter alia,* in therapeutic treatment and imaging of fibrosis and fibrotic tissue, including, but not limited to, liver (hepatic) fibrosis, cardiac fibrosis, kidney (renal) fibrosis, lung (pulmonary) fibrosis and skin fibrosis. The fibrosis may be early or advanced stage fibrosis. By way of example, the liver fibrosis may be cirrhosis. The fibrosis may be pre-cancerous fibrosis. Particular embodiments of the invention relating to therapeutic treatment of fibrosis contemplate combination treatments, wherein administration of the conjugate is in conjunction with one or more additional anti-fibrotic therapies, such as anti-fibrotic agents.

Suitable exemplary anti-fibrotic agents may include aminonitriles such as BAPN, primary amines such as ethylenediamine, hydrazine and phenylhydrazine, urea derivatives, copper chelating agents, and other small molecule, proteinaceous or nucleic acid-based agents that may be known to the skilled addressee. The identification and selection of suitable agents is well within the capabilities of the skilled addressee. The scope of the present disclosure is not limited to any one agent or type of agent and the preceding agents are provided by way of example only.

For such combination therapies, each component of the combination therapy may be administered at the same time, or sequentially in any order, or at different times, so as to provide the desired effect. Alternatively, the components may be formulated together in a single dosage unit as a combination product. When administered separately, it may be preferred for the components to be administered by the same route of administration, although it is not necessary for this to be so.

Biomolecule conjugates of the present invention find application, *inter alia,* in the imaging of atherosclerotic plaques and the therapeutic treatment and prevention of atherosclerosis. Accordingly, embodiments of the invention enable the treatment and prevention of atherosclerosis-related diseases and conditions such as myocardial infarction, coronary artery disease and peripheral vascular disease.

Particular embodiments of the invention relating to therapeutic treatment or prevention of atherosclerosis and atherosclerosis-related disease and conditions contemplate combination treatments, wherein administration of the conjugate is in conjunction with one or more additional anti-atherosclerotic therapies, such as anti-atherosclerotic agents. Suitable anti-atherosclerotic agents include, but are not limited to, statins, ACE inhibitors, niacin, fibrates (such as gemfibrozil and fenofibrate), calcium channel blockers and beta blockers.

Embodiments of the present invention also provide methods for imaging tumours, tumour cells, atherosclerotic tissue (such as plaques) and fibrotic tissue, wherein said imaging is facilitated or enhanced by the actions of biomolecule conjugates of the invention which increase access to tumours and fibrotic tissue of, and uptake by tumours and fibrotic tissue of, imaging agents or other detectable agents. Such imaging and detectable agents may be used, for example, in detecting, identifying, localizing and visualizing tumours, tumour cells, atherosclerotic tissue and fibrotic tissue in a subject. The nature and identity of suitable imaging agents will depend, for example, on the nature or type of tissue to be detected, identified, localized and/or visualized. The identification and selection of suitable agents is well within the capabilities of the skilled addressee, and include, for example, nanoparticulate imaging agents. A number of suitable nanoparticle-based detectable agents are well known to those skilled in the art, including for example iron oxide (IO) nanoparticles and IO nanoparticle-micelles. Other illustrative examples of suitable detectable agents include radio-isotopes, imaging dyes, alternative paramagnetic material or microbubbles. It will be understood by persons skilled in the art that the choice of detectable agent will depend on the method that will be employed for detection. Biomolecule conjugates of the invention and imaging or detectable agents may be administered at the same time, or sequentially, so as to provide the desired effect.

In accordance with the therapeutic and imaging aspects and embodiments of the invention described above, the biological conjugate or composition comprising the biological conjugate may further comprise further comprise a carrier. Suitable carriers will be familiar to persons skilled in the art, illustrative examples of which include polymeric nanoparticles, dendrimers, carbon nanotubes, gold nanoparticles, liposomes and micelles. In an embodiment disclosed herein, the carrier is a nanoparticle. Suitable nanoparticles will be familiar to persons skilled in the art, illustrative examples of which include poly(2-diisopropylaminoethyl methacrylate (PDPA) nanoparticles, IO nanoparticles and IO nanoparticle-micelles. Nanoparticles are currently being developed for biomedical applications such as sensing, bio-reactions and drug delivery. It is desirable that the functional cargo be preferentially retained in the nanoparticle either by using impermeable material or by conjugating the cargo to the nanoparticle shell. Conjugation offers some advantages, such as chemical control over the linking moieties, which can be pH, redox or enzyme responsive, while impermeable nanoparticles offer other advantages such as higher drug loading and responsiveness based on their shell properties, such as enzymatic degradation, and pH- or salt-induced swelling. Besides the retention of functional cargo, nanoparticles generally need a controlled surface for biomedical applications, such as stealth and targeting functionalities for site-specific drug delivery. By controlling the surface of the nanoparticle, the interaction between the particle and its surrounding environment is also controlled, and therefore specific functions that arise from the encapsulated or conjugated cargo can be localized to specific environments that are targeted by the nanoparticle. Therefore, cargo retention and proper localization are two further considerations for effective *in vivo* drug delivery.

Accordingly, aspects and embodiments of the present invention facilitate the use of, for example, a nanoparticle or other polymer-based platform for the *in vivo* targeted delivery of biological conjugates described herein to areas of abnormal extracellular matrix, such as in tumours, atherosclerotic tissue and fibrotic tissue. In an exemplary embodiment, the CSG peptide, or biomolecule conjugate according to the invention may be provided in, or linked to, IO nanoparticle micelles to facilitate efficient delivery of agents to the ECM. As exemplified herein, IO nanoparticle micelles targeted to the ECM home and accumulate in tumours more effectively than IO micelles targeted with the vessel homing peptide CREKA. Those skilled in the art will appreciate that nanoparticle and micelle-based delivery vehicles for CSG peptides and biomolecule conjugates of the present disclosure, optionally with a further therapeutic, detection or imaging agent, may be constructed using methods and protocols well known to those skilled in the art.

Any suitable amount or dose of a biomolecule conjugate of the present invention may be administered to a subject in need, depending on the application. For therapeutic applications, the therapeutically effective amount for any particular subject may depend upon a variety of factors including: the tumour or fibrosis being treated and the severity of the tumour or fibrosis; the activity of the conjugate employed; the composition employed; the age, body weight, general health, sex and diet of the subject; the time of administration; the route of administration; the rate of sequestration of the molecule or agent; the duration of the treatment; drugs used in combination or coincidental with the treatment, together with other related factors well known in medicine. One skilled in the art would be able, by routine experimentation, to determine an effective, non-toxic amount of protein conjugate to be employed.

The effective amount of conjugate may be between about 0.1 ng per kg body weight to about 100 µg per kg body weight, or typically between about 0.2 ng per kg body weight and about 10 µg per kg body weight. The effective amount may be, for example, about 0.2 ng, 0.4 ng, 0.6 ng, 0.8 ng, 1 ng, 1.5 ng, 2 ng, 2.5 ng, 3 ng, 3.5 ng, 4 ng, 4.5 ng, 5 ng, 5.5 ng, 6 ng, 6.5 ng, 7 ng, 7.5 ng, 8 ng, 8.5 ng, 9 ng, 9.5 ng, 10 ng, 11 ng, 12 ng, 13 ng, 14 ng, 15 ng, 16 ng, 17 ng, 18 ng, 19 ng, 20 ng, 25 ng, 30 ng, 35 ng, 40 ng, 45 ng, 50 ng, 55 ng, 60 ng, 65 ng, 70 ng, 75 ng, 80 ng, 85 ng, 90 ng, 95 ng, 100 ng, 150 ng, 200 ng, 250 ng, 300 ng, 350 ng, 400 ng, 450 ng, 500 ng, 550 ng, 600 ng, 650 ng, 700 ng, 750 ng, 800 ng, 850 ng, 900 ng, 950 ng, 1000 ng, 1100 ng, 1200 ng, 1300 ng, 1400 ng, 1500 ng, 1600 ng, 1700 ng, 1800 ng, 1900 ng or 2000 ng per kg body weight.

The skilled addressee will appreciate that among the factors determining the appropriate dose of conjugate to be administered will be the nature of the tumour or fibrosis homing peptide employed and the affinity, selectivity and/or specificity of that peptide for the particular tumour or fibrosis type to be treated.

The skilled addressee will also recognise that in determining an appropriate and effective dosage range for administration to humans based on the mouse studies exemplified herein, dose escalation studies would be conducted. The skilled addressee would therefore appreciate that the above mentioned doses and dosage ranges are exemplary only based on the doses administered in the mouse studies exemplified herein, and the actual dose or dosage range to be employed in humans may be varied depending on the results of such dose escalation studies. Based on the data exemplified herein, the appropriate and effective dose or dosage range to be administered to humans can be determined by routine optimisation, without undue burden or experimentation.

Biomolecule conjugates as disclosed herein may be administered to subjects, or contacted with cells, in accordance with aspects and embodiments of the present invention in the form of pharmaceutical compositions, which compositions may comprise one or more pharmaceutically acceptable carriers, excipients or diluents suitable for *in vivo* administration to subjects, and optionally one or more chemotherapeutic, immunotherapeutic, radiotherapeutic and/or anti-fibrotic agents. Where multiple agents are to be administered, each agent in the combination may be formulated into separate compositions or may be co-formulated into a single composition. If formulated in different compositions the compositions may be co-administered. By "co-administered" is meant simultaneous administration in the same formulation or in two different formulations via the same or different routes or sequential administration by the same or different routes. By "sequential" administration is meant a time difference of from seconds, minutes, hours or days between the administration of the two compositions. The compositions may be administered in any order, although in particular embodiments it may be advantageous for the peptide-protein conjugate to be administered prior to the chemotherapeutic, immunotherapeutic or radiotherapeutic agent.

Compositions may be administered to subjects in need thereof via any convenient or suitable route such as by parenteral (including, for example, intraarterial, intravenous, intramuscular, subcutaneous), topical (including dermal, transdermal, subcutaneous, etc), oral, nasal, mucosal (including sublingual), or intracavitary routes. Thus compositions may be formulated in a variety of forms including solutions, suspensions, emulsions, and solid forms and are typically formulated so as to be suitable for the chosen route of administration, for example as an injectable formulations suitable for parenteral administration, capsules, tablets, caplets, elixirs for oral ingestion, in an aerosol form suitable for administration by inhalation (such as by intranasal inhalation or oral inhalation), or ointments, creams, gels, or lotions suitable for topical administration. The preferred route of administration will depend on a number of factors including the tumour to be treated and the desired outcome.

The most advantageous route for any given circumstance can be determined by those skilled in the art. For example, in circumstances where it is required that appropriate concentrations of the desired agent are delivered directly to the site in the body to be treated, administration may be regional rather than systemic. Regional administration provides the capability of delivering very high local concentrations of the desired agent to the required site and thus is suitable for achieving the desired therapeutic or preventative effect whilst avoiding exposure of other organs of the body to the compound and thereby potentially reducing side effects.

In general, suitable compositions may be prepared according to methods known to those of ordinary skill in the art and may include a pharmaceutically acceptable diluent, adjuvant and/or excipient. The diluents, adjuvants and excipients must be "acceptable" in terms of being compatible with the other ingredients of the composition, and not deleterious to the recipient thereof. Pharmaceutical carriers for preparation of pharmaceutical compositions are well known in the art, as set out in textbooks such as Remington's Pharmaceutical Sciences, 20^{th}Edition, Williams& Wilkins, Pennsylvania, USA. The carrier will depend on the route of administration, and again the person skilled in the art will readily be able to determine the most suitable formulation for each particular case.

For administration as an injectable solution or suspension, non-toxic parenteral acceptable diluents or carriers can include Ringer's solution, medium chain triglyceride (MCT), isotonic saline, phosphate buffered saline, ethanol and 1 ,2 propylene glycol. Some examples of suitable carriers, diluents, excipients and adjuvants for oral use include peanut oil, liquid paraffin, sodium carboxymethylcellulose, methylcellulose, sodium alginate, gum acacia, gum tragacanth, dextrose, sucrose, sorbitol, mannitol, gelatine and lecithin. In addition these oral formulations may contain suitable flavouring and colourings agents. When used in capsule form the capsules may be coated with compounds such as glyceryl monostearate or glyceryl distearate which delay disintegration.

Adjuvants typically include emollients, emulsifiers, thickening agents, preservatives, bactericides and buffering agents.

Methods for preparing parenteral administrable compositions are apparent to those skilled in the art, and are described in more detail in, for example, Remington's Pharmaceutical Science, 15th ed., Mack Publishing Company, Easton, Pa. The composition may incorporate any suitable surfactant such as an anionic, cationic or non-ionic surfactant such as sorbitan esters or polyoxyethylene derivatives thereof. Suspending agents such as natural gums, cellulose derivatives or inorganic materials such as silica ceoussilicas, and other ingredients such as lanolin, may also be included.

Solid forms for oral administration may contain binders acceptable in human and veterinary pharmaceutical practice, sweeteners, disintegrating agents, diluents, flavourings, coating agents, preservatives, lubricants and/or time delay agents. Suitable binders include gum acacia, gelatine, corn starch, gum tragacanth, sodium alginate, carboxymethylcellulose or polyethylene glycol. Suitable sweeteners include sucrose, lactose, glucose, aspartame or saccharine. Suitable disintegrating agents include com starch, methylcellulose, polyvinylpyrrolidone, guar gum, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propylparaben or sodium bisulphite. Suitable lubricants include magnesium stearate, stearic acid, sodium oleate, sodium chloride or talc. Suitable time delay agents include glyceryl monostearate or glyceryl distearate.

Liquid forms for oral administration may contain, in addition to the above agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides or mixtures thereof.

Suspensions for oral administration may further comprise dispersing agents and/or suspending agents. Suitable suspending agents include sodium carboxymethylcellulose, methylcellulose, hydroxypropylmethyl-cellulose, poly-vinyl-pyrrolidone, sodium alginate or acetyl alcohol. Suitable dispersing agents include lecithin, polyoxyethylene esters of fatty acids such as stearic acid, polyoxyethylene sorbitol mono- or di-oleate, -stearate or -laurate. Polyoxyethylene sorbitan mono-or di-oleate, -stearate or -laurate and the like.

Emulsions for oral administration may further comprise one or more emulsifying agents. Suitable emulsifying agents include dispersing agents as exemplified above or natural gums such as guar gum, gum acacia or gum tragacanth.

Compositions of the invention may be packaged and delivered in suitable delivery vehicles which may serve to target or deliver the peptide-protein conjugate, and optionally one or more additional agents to the required tumour site and/or to facilitate monitoring of tumour uptake by, for example MRI imaging or other imaging techniques known in the art. By way of example, the delivery vehicle may comprise liposomes, or other liposome-like compositions such as micelles (e.g. polymeric micelles), lipoprotein-based drug carriers, microparticles, nanoparticles, or dendrimers.

Liposomes may be derived from phospholipids or other lipid substances, and are formed by mono- or multi-lamellar hydrated liquid crystals dispersed in aqueous medium. Specific examples of liposomes used in administering or delivering a composition to target cells are DODMA, synthetic cholesterol, DSPC, PEG-cDMA, DLinDMA, or any other non-toxic, physiologically acceptable and metabolisable lipid capable of forming liposomes. The compositions in liposome form may contain stabilisers, preservatives and/or excipients. Methods for preparing liposomes are well known in the art, for example see Methods in Cell Biology, Volume XIV, Academic Press, New York, N.Y. (1976), p. 33 ff.. Biodegradable microparticles or nanoparticles formed from, for example, polylactide (PLA), polylactide-co-glycolide (PLGA), and epsilon-caprolactone (έ-caprolactone) may be used.

Other means of packaging and/or delivering conjugates, and optionally one or more additional agents, in order to monitor tumour uptake will also be well known to those skilled in the art.

As described and exemplified herein, the present inventors have also elucidated, for the first time, the ability of a peptide comprising or consisting of the sequence set forth in SEQ ID NO: 1 to target the ECM of a variety of tumour types, atherosclerotic tissue and fibrotic tissue.

Accordingly, an aspect of the invention provides the use of a peptide comprising or consisting of the sequence set forth in SEQ ID NO:1 for the detection and/or localisation of tumours, atherosclerosis or fibrosis. Also provided herein is a peptide comprising or consisting of the sequence set forth in SEQ ID NO:1 for use in a method for detecting and/or localising a tumour, atherosclerotic tissue or fibrotic tissue, comprising exposing tissue, or a biological sample comprising tissue, to a peptide comprising or consisting of the sequence set forth in SEQ ID NO:1.

To achieve this, the peptide may be conjugated to, combined with, or administered in conjunction with a suitable compound or molecule for detecting and/or imaging tissue. Therefore, also provided herein is an imaging agent comprising a peptide linked to a detectable label or agent, wherein the peptide comprises or consists of sequence set forth in SEQ ID NO: 1. Such imaging agents may be used, for example, in detecting, identifying and localizing tumours, tumour cells and fibrotic tissue in a subject. The identification and selection of suitable detectable labels, agents and other compounds and molecules is well within the capabilities of the skilled addressee, and include, for example, nanoparticles. A number of suitable nanoparticle-based detectable agents are well known to those skilled in the art, including for example iron oxide (IO) nanoparticles and IO nanoparticle-micelles. Other illustrative examples of suitable detectable agents include radio-isotopes, imaging dyes, alternative paramagnetic material or microbubbles.

Suitable detectable labels and agents will be known to persons skilled in the art, illustrative examples of which include a radio-isotope, an imaging dye, a paramagnetic material or a microbubble. It will be understood by persons skilled in the art that the choice of detectable label or agent will depend on the method that will be employed to detect the imaging agent. For example, where the imaging agent is to be used to detect tumours, atherosclerotic plaques or areas of fibrosis *in vitro* or *ex vivo,* it may be desirable to use an imaging dye such as a fluorophore. Suitable fluorophores will be known to persons skilled in the art, illustrative examples of which include fluorescein (FITC), Cy3, Cy3.5, Cy5 and Cy3.5. Where the imaging agent is to be used to detect tumours, atherosclerotic plaques or areas of fibrosis *in vivo,* it may be desirable to use a contrasting agent such as a radiolabel or radio-isotope. In some embodiments, more than one detectable label or agent can be used.

The detectable label or agent can be linked to the peptide by any suitable method of conjugation. Suitable methods of conjugating the detectable label or agent to the polypeptide disclosed herein will be known to persons skilled in the art. The choice of conjugation method may depend on the detectable label or agent to be employed.

In some embodiments, methods of conjugating the detectable label or agent to the peptide may require a linker to be attached to the N-terminus or C-terminus of the peptide of SEQ ID NO: 1 or variant thereof to facilitate conjugation. Suitable linkers will be known to persons skilled in the art, illustrative examples of which include peptides and polypeptides comprising N-terminal LPETG, N-terminal ACPP-alkyne and C-terminal GGG, or any combination thereof.

In some embodiments, it may be desirable to attach the detectable label or agent to a complexing agent to maximise retention of the detectable label or agent to the imaging agent and thereby minimise loss or degradation of the detectable label or agent from the imaging agent, in particular under physiological conditions. Suitable complexing agents will be known to persons skilled in the art, an illustrative example of which is 1,4,7,10-tetraazacyclododecane-1,4,7,10-tetraacetic acid (DOTA).

Peptides comprising or consisting of the sequence of SEQ ID NO:1 and imaging agents containing said peptides may further comprise, or be linked to, a carrier. Suitable carriers will be familiar to persons skilled in the art, illustrative examples of which include polymeric nanoparticles, dendrimers, carbon nanotubes, gold nanoparticles, liposomes and micelles. In an embodiment disclosed herein, the carrier is a nanoparticle. Suitable nanoparticles will be familiar to persons skilled in the art, illustrative examples of which include poly(2-diisopropylaminoethyl methacrylate (PDPA) nanoparticles, IO nanoparticles and IO nanoparticle-micelles.

Accordingly, aspects and embodiments of the present invention facilitate the use of, for example, a nanoparticle or other polymer-based platform for the delivery of the CSG peptide to facilitate the detection, localisation, and imaging of ECM and areas of abnormal ECM, such as in tumours, atherosclerotic tissue and fibrotic tissue. In an exemplary embodiment, the CSG peptide may be provided in, or linked to, IO nanoparticle micelles to facilitate efficient delivery of agents to the ECM. The CSG peptide may or may not be conjugated to a cytokine as described hereinbefore. As exemplified herein, IO nanoparticle micelles targeted to the ECM home and accumulate in tumours more effectively than IO micelles targeted with the vessel homing peptide CREKA. Those skilled in the art will appreciate that nanoparticle and micelle-based delivery vehicles for CSG peptides (and biomolecule conjugates of the present disclosure), optionally with a further therapeutic, detection or imaging agent, may be constructed using methods and protocols well known to those skilled in the art.

Moreover, CSG-conjugated carriers such as IO nanoparticle micelles and the like can be used as delivery vehicles for the delivery of therapeutic agents to areas of abnormal ECM, including tumours, atherosclerotic tissue and fibrotic tissue. The therapeutic agent may be a biomolecule conjugate of the present invention, and/or any other suitable anti-tumour, anti-atherosclerotic or anti-fibrotic agent.

The tumour may be a lung tumour, such as small cell lung cancer or non-small cell lung cancer; a pancreatic tumour, such as an insulinoma; a bladder tumour; a kidney tumour; a brain tumour, such as a glioblastoma or medulloblastoma; a neuroblastoma; a head and neck tumour; a thyroid tumour; a cervical tumour; a prostate tumour; a testicular tumour; an ovarian tumour; an endometrial tumour; a rectal and colorectal tumour; a stomach tumour; an esophageal tumour; a skin tumour, such as a melanoma or squamous cell carcinoma; an oral tumour including squamous cell carcinoma; a liver tumour, including human hepatocellular carcinona (HCC); a lymphomas; a sarcomas, including osteosarcoma, liposarcoma and fibrosarcoma. The fibrosis may be liver fibrosis, cardiac fibrosis, kidney fibrosis, lung fibrosis or skin fibrosis. The fibrosis may be pre-cancerous fibrosis. The atherosclerotic tissue may be a fibroatheroma or an atherosclerotic plaque.

Embodiments of the invention described herein employ, unless otherwise indicated, conventional molecular biology and pharmacology known to, and within the ordinary skill of, those skilled the art. Such techniques are described in, for example, "Molecular Cloning: A Laboratory Manual", 2nd Ed., (ed. by Sambrook, Fritsch and Maniatis) (Cold Spring Harbor Laboratory Press: 1989); "Nucleic Acid Hybridization", (Hames& Higgins eds. 1984); Oligonucleotide Synthesis" (Gait ed,, 1984); Remington's Pharmaceutical Sciences, 17th Edition, Mack Publishing Company, Easton, Pennsylvania, USA.; "The Merck Index", 12th Edition (1996), Therapeutic Category and Biological Activity Index,- and "Transcription & Translation", (Hames & Higgins eds. 1984).

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

The present invention will now be described with reference to the following specific examples, which should not be construed as in any way limiting the scope of the invention.

### EXAMPLES

### General methods

Dextran coated iron oxide encapsulated with 18:0 PEG2000 PE micelle formulation and doxorubicin-micelles were made in the laboratory. 4T1 murine breast carcinoma is an orthotopic implant model by inoculating of 5 × 10⁵ cells in the mammary fat pad of syngeneic BALB/c mice. RIP1-Tag5 insulinoma model (on C3H background) contains the oncogene SV40 Large T antigen (Tag; RIP, rat insulin gene promoter) that is exclusively expressed in β cells of the pancreas resulting in pancreatic tumours of neuroendocrine origin. ALB-Tag HCC (in C3H background) contains the oncogene SV40 Large T antigen (Tag) that is exclusively expressed in hepatocytes under the control of the albumin (ALB) promoter, resulting in rapid (non-metastatic) HCC tumour progression.

### Example 1 - CSG specifically targets tumour ECM

Fluorescein-labelled (FAM)-CSG (CSGRRSSKC; SEQ ID NO:1) (100 µL of 1mM stock made in sterile 1× PBS) was injected into the tail vein of tumour-bearing mice (hepatocellular carcinoma or breast carcinoma, in C3H and BALB/c strains, respectively). Tissue was harvested without perfusion 1 hr after injection and peptide accumulation observed under UV illumination. As shown in Figure 1A, FAM-CSG accumulates in both tumour types. Homing was specific to tumours, with only clearance organs (intestines, kidneys) showing limited accumulation of FAM-CSG.

Fresh, untreated samples of human breast carcimoma were dipped, 1 hr post surgery, in 20 µM FAM-CSG for 1 hr, followed by 3 x15 min washes with 1× PBS. As shown in Figure 1B, the FAM-CSG bound specifically to the tumour tissue and not to normal or marginal tissue. Pre-incubation with an excess 1 mg of unlabeled CSG peptide abolished the FAM-CSG specific penetration and accumulation in tumours.

Figure 1B also demonstrates that this binding specificity is unique to CSG. A control fluorescein-labelled (FAM)-ARA peptide, ARALPSQRSR (SEQ ID NO:5) (1 mM, 100 µL) showed no binding to the human breast carcinoma tissue. This absence of binding of FAM-ARA was confirmed using anti-fluorescein antibody (data not shown). Similar results were observed with murine breast carcinoma (4T1 tumour), hepatocellular carcinoma and insulinoma (data not shown).

The inventors also determined the site of localisation of CSG within tumours, and found that CSG co-localises with two known ECM markers, collagen-1 and nidogen-1, in murine insulinoma, murine hepatocellular carcinoma, human breast carcinoma and human hepatocellular carcinoma. Following incubation of whole tissue (for mouse insulinoma and human breast cancer) or 8 µm tissue cross section (for mouse and human HCC) with FAM-CSG peptide, tissue cross sections were co-stained with 0.3 µg in 100 µL of anti-nidogen-1 (Rat anti-monoclonal mouse nidogen-1, Millipore) or anti-collagen-1 (Rabbit polyclonal collagen 1, Abcam) markers of ECM, for 1 hr at room temperature. Stained sections were washed 3 × with 1×TBS buffer. Tissue sections were re-incubated with donkey anti-Rat or Rabbit IgG (H+L) secondary antibody Alexa 594 (0.4 µg in 100 µL for 30 min at room temperature), followed by 3 × wash with 1×TBS buffer. Co-localisation of CSG with these ECM markers highlights the specificity of CSG binding to tumour ECM (Figure 2). Co-localisation was not observed in normal healthy tissue. These results demonstrate that CSG specifically targets tumour ECM.

The inventors also tagged (conjugated) iron oxide (IO) nanoparticle micelles with CSG. Dextran-coated iron oxide nanoparticles were encapsulated in biocompatible pegylated lipids (PEG2000 PE) that either contain conjugated fluorescein (FAM), FAM-CREKA or FAM-CSG, producing IO micelles as neutral nanoparticles between 25-30 nm in diameter. Briefly, 10 mg dextran was added dropwise to 10 mg iron oxide nanoparticles in 20 mL 0.5 M NaOH and sonicated with a probe sonicator for 1hr at 50 Hz. Coated nanoparticles were then dialyzed using a 10,000 MW Dialysis membrane (Sigma-Aldrich) for 24 h in 1.5 L distilled water to remove excess dextran. The nanoparticle suspension was kept in an oven at 120 °C for 1 hr and dried completely. The dried nanoparticles were dispersed and kept in hexane. DSPE-PEG2000 (50 mg, 71.29 mmol, Avanti Polar Lipids, Inc.) conjugated to fluorescein or FAM-labelled peptides was dissolved in 4 mL chloroform, and 1mL nanoparticles (6 mg Fe) was added. This suspension was injected into deionized water, stirred at 80 °C for 10 minutes, with a speed of 2 µl/sec. The mixture was stirred for one hour, and upon evaporation of the organic solvents, the nanoparticles were encapsulated in the core of phospholipidic micelles. Empty phospholipid micelles were removed by ultracentrifugation (42000 g; RT; 20 min). The pellet, containing the IO nanoparticle-micelles, was redispersed in PBS pH 7.4 by gently shaking.

Insulinoma-bearing mice were intravenously injected with 100 µL of 1 mM fluorescein-labelled untargeted IO nanoparticle micelles, CREKA-tagged IO nanoparticle micelles, or CSG-tagged nanoparticle IO micelles. Tumours were harvested after heart perfusion and imaged ex vivo by MRI and microscopic analysis. The MRI scan shows increased accumulation of CSG-tagged IO nanoparticle micelles in injected tumours shown by T2* mapping (Figure 3, top), T2 relaxation (data not shown) and histological detection of antibody against FAM (anti-FITC ab) (Figure 3, bottom) when com pared to both the untargeted IO micelles and the CREKA-tagged micelles.

### Example 2 - TNFα-CSG

Mature murine TNFα (SEQ ID NO:6) with or without a C-terminal conjugated peptide CSGRRSSKC (SEQ ID NO:1) connected via a GGG linker, was cloned into *Xho*I/*Bam*H1 sites of the vector pET-44a (Novagen) to express a soluble fusion protein with N-terminal Nus•Tag/His•Tag. Briefly, after isopropyl-β-d-glactopyranoside (IPTG) induction overnight at 25°C (TNFα), cultures were centrifuged, resuspended in lysis buffer (50 mM NaH₂PO₄, 300 mM NaCl, 10 mM imidazole, 1 mM DTT, 1mM PMSF, 1 mM EDTA, 1% Triton-XlOO, pH 8.0), sonicated, and purified using Ni-NTA beads (Qiagen) following the manufacturer's instructions. Nus·Tag/His·Tag was cleaved with tobacco etch virus (TEV) protease overnight at 4°C (TNFα). Recombinant proteins from cleavage reactions were dialyzed overnight in 3 × PBS and re-purified twice using Ni-NTA beads. Purity was assessed on Coomassie brilliant blue stained protein gels (Figure 4A).

Bioactivity of TNFα-CSG was assessed *in vitro,* specifically by incubating macrophage cell line, J774, on matrigel attached with TNFα-CSG (0.1 mmol). Incubation of the cells from 1 to 16 hrs results in the secretion of MMP-2 and MMP-9, proteases known to degrade ECM (Figure 4B).

Binding specificity of TNFα-CSG to tumours *in vivo* was assessed by detecting fluorescein-labelled TNFα-CSG and native unconjugated TNFα following 130 µg intravenous injection (30 min circulation). As shown in Figure 4C, TNFα-CSG specifically targeted RIP-Tag insulinoma and hepatocellular carcinoma in mice, displaying limited binding to normal pancreas, liver, heart and kidney. Unconjugated native TNFα has limited affinity to tumour tissue.

To determine potential toxicity effects of TNFα-CSG, the inventors then administered TNFα-CSG intravenously to mice (n=6) at a dose ranging from 0.5-10 µg daily, to a total of 8 injections. TNFα-CSG is non-toxic at these doses and frequency. In comparison all mice (n=6) died following only two daily intravenous injections of 2 µg native TNFα.

### Example 3 - Immune cell infiltration following TNFα-CSG treatment of tumours

Mice bearing 4T1 breast carcinoma, when tumour size reached 500 mm³, were treated with 0.5 and 2 µg TNFα-CSG or native TNFα (in 100 µL) by daily intravenous injection for 5 days. FACS quantification of CD4+ and CD8+ T cells and infiltrating macrophages (CD11b+/ CD68+/F4/80+) harvested in whole tumours is presented in Figure 5.

Quantitative analysis of these tumours shows the increase in immune cell infiltration is most effective in tumours treated with 2 µg TNFα-CSG (*P*< *0.02).* As noted in Example 2, native TNFα at 2 µg is toxic (all mice died after receiving 2 doses of 2 µg TNFα). A lower dose of native TNFα (0.5 µg) is not effective compared to 0.5 µg TNFα-CSG (Figure 5).

Mice bearing RIP-Tag insulinoma at 25 weeks of age were treated with 2 and 5 µg TNFα-CSG (in 100 µL) by daily intravenous injection for 5 days. Figure 6 presents microscopic evaluation of CD4+ and CD8+ T cells and circulatory CD11b+ macrophages detected by immunofluorescence staining of tissue cross sections. Quantitative analysis of these tumours shows a significant increase in immune cell infiltration in TNFα-CSG treated tumours (*P*< *0.01)* at both 2 and 5 µg doses, in comparison to the control groups.

Mice bearing hepatocellular carcinoma at 10 weeks of age were treated with 2 µg TNFα-CSG or control CSG peptide (in 100 µL) by daily intravenous injection for 5 days. The experiment was also conducted on mice with normal, healthy livers. FACS quantification of CD4+ and CD8+ T cells and infiltrating macrophages (CD11b+/CD68+) in whole tumours is shown in Figure 7. Quantitative analysis of these tumours show at least 3 fold increase in immune cell infiltration compared to CSG-treated tumours *(P*<*0.05).* The treatment has no effect on normal liver.

CD45+ leukocytes isolated from 4T1 tumours in BALB/c mice following treatment with 10 µg TNFα-CSG were found to express a number of proteases (uPA, MMP-2, MMP-3, MMP9, MMP-12, MMP-14, cathepsin B, Cathepsin L and ADAM-9) at significantly higher levels (3 to 20-fold higher), relative to the expression of hypoxanthine-guanine phosphoribosyl transferase (HPRT), than the same cell type isolated from tumours in mice treated with 0.8 µg CSG (Figure 8). In both groups of mice administration of the CSG or TNFα-CSG was by intravenous injection daily for five days.

The inventors also generated a conjugate between mature murine interferon gamma (IFNy) (SEQ ID NO: 10) and CSG in a similar manner as for TNFα-CSG, namely connecting the CSG peptide sequence of SEQ ID NO:1 to the C-terminal of IFNγ via a GGG linker. Mature murine IFNγ (SEQ ID NO: 10) with or without a C-terminal conjugated peptide CSGRRSSKC (SEQ ID NO:1) connected via a GGG linker, was cloned into *Xho*I/*Bam*H1 sites of the vector pET-44a (Novagen) to express a soluble fusion protein with N-terminal Nus·Tag/His·Tag. Briefly, after isopropyl-β-d-glactopyranoside (IPTG) induction for 6 hrs at 30°C (IFNy), cultures were centrifuged, resuspended in lysis buffer (50 mM NaH₂PO₄, 300 mM NaCl, 10 mM imidazole, 1 mM DTT, 1mM PMSF, 1 mM EDTA, 1% Triton-XlOO, pH 8.0), sonicated, and purified using Ni-NTA beads (Qiagen) following the manufacturer's instructions. Nus·Tag/His·Tag was cleaved with tobacco etch virus (TEV) protease overnight at 4°C (IFNy). Recombinant proteins from cleavage reactions were dialyzed overnight in 3 × PBS and re-purified twice using Ni-NTA beads.

Mice bearing RIP-Tag insulinomas, at 25 weeks of age, were treated with 5 µg IFNγ-CSG or controls (native IFNγ and CSG peptide control individually) by daily intravenous injection for 5 days. Microscopic evaluation of CD4+ T cells detected by immunofluorescence staining of tissue cross sections, demonstrated that, similar to TNFα-CSG, IFNγ-CSG treatment resulted in a significant increase in immune cell infiltration compared to the control groups, with immune cells aggregating on the ECM (data not shown).

### Example 4 - Effect of TNFα-CSG on tumour ECM

As evidenced by analysis of the ECM components collagen-1, laminin and nidogen-1, intravenous injection of TNFα-CSG at a dose of 2 µg or 5 µg daily for five days to mice having a RIP-Tag insulinoma, specifically reduces tumour ECM content (Figure 9). Quantitative analysis of ECM positive for collagen-1, laminin and nidogen 1, excluding basement membrane shows a significant reduction in ECM content *(P*<*0.05).* This effect on tumour ECM was restricted to tumours; ECM content in normal pancreas remained unaltered.

Further, mapping of tumour stiffness (4T1 breast carcinoma and RIP-Tag insulinoma) by optical coherence tomography/ micro-elastography revealed that TNFα-CSG treatment (2 µg/day × 4 total injections) reduced tumour stiffness well below 100 kPa, compared to frequent stiffness "hotspots" in control tumours which exceed 100 kPa (Figure 10). Frequency distribution of this data revealed that tissue stiffness (stiffness >100 kPa) and stiffness variance are significantly reduced in response to TNFα-CSG treatment (Figure 11).

The inventors then evaluated vessel perfusion in RIP-Tag insulinoma and 4T1 breast carcinoma from mice treated with TNFα-CSG (2 µg/day ×5), as measured by CD31: lectin ratio following 10 min intravenous circulation of tomato green lectin. As shown in Figure 12A, in TNF-α CSG treated tumours, the lectin-positive staining significantly correlated with actual vessel density (CD31: lectin ratio close to 1). In contrast, control treated tumours were poorly perfused and did not completely match actually vessel density (CD31:lectin ratio >1). Vessels in TNFα-CSG treated tumours were also significantly wider than in control treated tumours, and hence improved in perfusion (Figure 12B & C). Similar results were obtained with mice bearing ALB-Tag hepatocellular carcinoma (data not shown).

### Example 5 - Effect of TNFα-CSG on tumour uptake

As a result of the reduced tumour ECM content and increased tumour vessel perfusion (Example 4), the inventors then evaluated the ability of tumours treated with TNFα-CSG to take up various reagents. Mice bearing 4T1 tumour were treated with 2 µg TNFα-CSG or CSG control peptide by intravenous injection for five days. Following this, mice were intravenously injected with 100 µL of 0.9% Evans blue solution and allowed to circulate for 30 min. Tumours were then harvested and photographed. As shown in Figure 13, TNFα-CSG treated tumours are more permeable to Evans blue dye than control-treated tumours.

Reduced tumour ECM and stiffness, as well as improved perfusion was also shown to result in enhanced tumour uptake of a nano-imaging contrast agent. Specifically, insulinoma-bearing mice were treated with 2-10 µg TNFα-CSG or CSG control peptide by intravenous injection daily for five days. Following this, mice were intravenously injected with 100 µL of 1 mM iron oxide (IO) micelles (30 nm diameter). Tumours were harvested after 6 hr circulation and imaged by MRI and microscopic analysis of tissues mounted in 2 % agarose. As shown in Figure 14, MRI scanning showed increased accumulation of FITC-labelled IO micelles in TNFα-CSG treated tumours (2 µg dose) shown by T2* and T2 relaxation. The loss of signal (i.e. T2 relaxation time msec) is significantly lower, representing greater iron oxide micelle accumulation. This accumulation was confirmed by immunostaining analysis of tissue cross sections using anti-FITC antibody.

4T1 breast carcinoma-bearing mice were treated with 2 µg TNFα-CSG or CSG control peptide by intravenous injection daily for 5 days. Mice were then injected with 100 µL of 1 mM doxorubicin-micelles (200 nm in size) and tissues including tumours, spleen and liver were collected for analysis. Microscopic evaluation shown in Figure 15 indicates strong traces of doxorubicin (autofluorescence) in TNFα-CSG treated tumours, comparable to the non-specific uptake of doxorubicin micelles in spleen and liver.

### Example 6 - Anti-tumourigenic effects of TNFα CSG

The inventors have also discovered that TNFα-CSG has anti-tumorigenic effects, as evidenced by reduced tumour growth and reduced cell proliferation. 4T1 tumour-bearing mice were treated with 2 µg TNFα-CSG or control CSG peptide 4 days after inoculation with 5 × 10⁵ 4T1 cells. Each mouse received a total of 6 injections in 2 weeks. As shown in Figure 16A, tumour size and weight were significantly reduced in the TNFα-CSG treated mice. Further, microscopic evaluation of whole tumours for the cell proliferation marker Ki67⁺ indicated a significant reduction in tumour cell proliferation in TNFα-CSG treated tumours (Figure 16B). FACs quantification of infiltrating T cell populations positive for CD8⁺ cytotoxic T cells expressing granzyme B and CD107a and positive for CD4⁺ T cells expressing FoxP3 and CD25 demonstrated a significantly higher ratio of cytotoxic T cells to immune suppressor T cells in TNFα-CSG treated tumours (Figure 16C).

The inventors have also surprisingly found that treatment with TNFα-CSG reduced secondary metastasis of 4T1 breast tumours. 4T1 tumour-bearing mice were treated with 10 µg TNFα-CSG or CSG control peptide daily by intravenous injection once primary tumours reached at least 500 mm³, up to a total of eight injections. 4T1 cells in blood, lung, liver and lymph nodes were harvested once the primary tumours reached 2,000 mm³. Cell suspensions from these tissues were cultured in 6-thioguanine - containing media to select for 4T1 cells that are resistant to 6-thioguanine. Colonic metastasis of methylene blue stained cells were counted. It is known that the lung is the primary site of metastasis of breast tumours. However as shown in Figure 17A, quantitative analysis of cell density/tissue (% mean ± SD) indicates significantly reduced lung metastasis in TNFα-CSG treated breast tumour-bearing mice. Similarly, the colonic metastasis counts in liver and lymph nodes were lower in the TNFα-CSG treated group.

This reduced metastasis correlated with significantly reduced hypoxia in primary tumours (Figure 17B). The TNFα-CSG treatment also resulted in enlarged tumour centres that were cleared of tumour burden (negative for hypoxia, blood vessels and cell proliferation marker ki67) (Figure 17C).

Whole microscopic images of 4T1 tumours stained with a hypoxia marker (Pimonidazole Hydrochloride) , following treatment of tumour-bearing mice with 10 µg TNFα-CSG for five days, showed large areas at the centre of the tumour devoid of tumour cells (Figure 18A). Similar results were observed with RIP-Tag tumours following treatment of tumour-bearing mice with 5 µg TNFα-CSG treatment for five days stained with immune cell markers (CD4 and CD8) and lectin (Figure 18B).

Survival studies were conducted using mice bearing advanced insulinoma, at 24 weeks of age, treated with 5 µg TNFα-CSG or 0.8 µg CSG daily for five days by intravenous injection. As shown in Figure 19, survival of TNFα-CSG treated mice was significantly enhanced compared to control, CSG-treated, mice.

### Example 7 - Binding of CSG to fibrotic tissue in the liver

In mice fed a choline deficient diet that triggers development of fibrosis, cirrhosis and hepatocellular carcinoma, the inventors have shown that CSG effectively binds fibrotic tissue at all stages (Figure 20A). Mice (C57BL/6 strain) were fed a choline deficient diet for 4 weeks (early liver fibrosis), 16 weeks (advanced fibrosis-cirrhosis) or 28 weeks (advanced HCC), after which liver tissue was examined. Acetone-fixed fresh frozen 8 µm issue cross sections were incubated with either 1 µM FAM-CSG or 5 µM FAM-ARA for 30 min in 1 × PBS buffer at room temperature. FAM-CSG or FAM-ARA binding were confirmed using anti-FITC-HRP antibody staining. The FAM-CSG staining co-localised with staining for the ECM marker nidogen-1 (Figure 20B), demonstrating that the ECM abnormality associated with CSG is expressed from an early fibrotic stage.

### Example 8 - Binding of CSG to atherosclerotic plaque

To determine whether CSG also recognises fibroatheroma, healthy aorta from wild type C57BL/6 mice and aorta from ApoE null mice were incubated with 20 nmol/ml fluorescein-labelled FAM-CSG or ARA control. As shown in Figure 21A, FAM-CSG specifically bound to, and accumulated in, plaques from the ApoE null mice. Immunostaining of aorta containing plaque demonstrates that in vivo accumulation of CSG (following 1 hr intravenous injection of 100 µl 1 mM FAM-CSG) co-localises with the ECM marker laminin (Figure 21B). Occluded arteries obtained from a human patient with occlusive peripheral vascular disease after limb amputation also showed specific binding of CSG to the occluded arteries and specific accumulation of CSG in plaques (Figure 21C).

### Example 9 - Effect of TNFα-CSG on atherosclerotic plaque

The inventors then evaluated the effect of TNFα-CSG (see Example 2) on plaque formation in ApoE null mice. Aging ApoE null mice were treated with 10 µg TNFα-CSG or 0.8 µg CSG peptide daily for five days by intravenous injection. Tissue, including aorta and plasma, were collected from euthanised animals at 70 weeks of age. Plasma samples were collected after 1 and 10 weeks of therapy and assessed for markers of (i) circulating cholesterol (total cholesterol, HDL and LDL), (ii) cardiac necrosis (troponin) and (iii) liver damage (alanine transaminase, ALT and aspartate transaminase, AST). As shown in Figure 22A, quantification of plaque positive area indicates that TNFα-CSG therapy significantly reduced plaque burden. This correlated with an increase in circulating HDL levels and a reduction in LDL in the TNFα-CSG treated mice 10 weeks after administration of the TNFα-CSG (Figure 22B). This treatment with TNFα-CSG did not elevate cardiac or liver toxicity, as demonstrated by measured levels of plasma troponin, alanine transaminase (ALT) and aspartate aminotransferase (AST) (Table 1 below).

**Table 1**

| | Control (1 week) | TNFα-CSG (1 week) | Control (10 weeks) | TNFα-CSG (10 weeks) |
|---|---|---|---|---|
| Troponin (ng/L) | 13.7 (6.1-42.4) | 15.3 (10.7-30.2) | 20.0 (16.0-42.9) | 15.1 (5.7-24.0) |
| ALT (U/L) | 37.7 (18.3-59.6) | 33.3 (15.4-115.5) | 34.6 (27.5-84.8) | 26.0 (18.5-79.8) |
| AST (U/L) | 92.0(67.8-110.8) | 106 (74.0-195.5) | 99.5 (59.0-179.3) | 81.5 (52.3-184.3) |

| | | | | |
|---|---|---|---|---|
| Median (interquartile range) figures shown. Data are non-significant by Mann Whitney U test. All P values >0.05. | | | | |

Further analysis of the effect of TNFα-CSG showed that the TNFα-CSG treatment of the ApoE null mice degraded ECM in plaque intima (as determined by reduced collagen I, collagen IV and laminin), reduced macrophage content (CD1 1b+) and increased the expression of activate endothelia (CD31+, endoglin+) in plaque intima (Figure 23).

## Claims

1. A biomolecule conjugate comprising a cytokine and a peptide comprising or consisting of the sequence set forth in SEQ ID NO: 1 or a conservative variant thereof.

2. A biomolecule conjugate according to claim 1, wherein the cytokine is an immunopotentiating cytokine that mediates a cellular immune response.

3. A biomolecule conjugate according to claim 2, wherein the immunopotentiating cytokine is TNFα or IFNγ.

4. A biomolecule conjugate according to any one of claims 1 to 3, wherein the peptide comprising or consisting of the sequence set forth in SEQ ID NO:1, or conservative variant thereof, is conjugated to the C-terminal end of the cytokine.

5. A biomolecule conjugate according to claim 4, wherein the peptide is conjugated to the C-terminal end of the cytokine via a linker sequence comprising one or more, two or more, or three or more, glycine (G) residues.

6. A pharmaceutical composition comprising a biomolecule conjugate according to any one of claims 1 to 3, or a polynucleotide encoding the same, wherein the composition further comprises one or more pharmaceutically acceptable carriers, adjuvants and/or excipients.

7. A pharmaceutical composition according to claim 6, further comprising one or more additional anti-tumorigenic, anti-atherosclerotic or anti-fibrotic agents.

8. A biomolecule conjugate according to any one of claims 1 to 5 or pharmaceutical composition according to claim 6 or 7 for use in a method for treating a condition associated with abnormal ECM.

9. A biomolecule conjugate according to any one of claims 1 to 5 or pharmaceutical composition according to claim 6 or 7 for use according to claim 8, wherein the treatment degrades the extracellular matrix (ECM) of tumour, atherosclerotic or fibrotic tissue or promotes or induces immune cell infiltration of a tumour, atherosclerotic tissue or fibrotic tissue.

10. A biomolecule conjugate or pharmaceutical composition for use according to claim 9, wherein the immune cells infiltrating the tumour or tissue express and release one or more proteases capable of degrading the tumour ECM.

11. A biomolecule conjugate or pharmaceutical composition for use according to claim 9 or 10, wherein the immune cells comprise T cells, macrophages and/or neutrophils.

12. A biomolecule conjugate or pharmaceutical composition for use according to claim 8, wherein the condition is a solid tumour, atherosclerosis or fibrosis.

13. A biomolecule conjugate or pharmaceutical composition for use according to claim 12, wherein treatment of the tumour with the conjugate or composition increases vessel perfusion in the tumour, increasing access of the one or more additional anti-cancer agents to the tumour and cancerous cells therein and thereby improving efficacy of said anti-cancer agents.

14. A biomolecule conjugate or pharmaceutical composition for use according to claim 12 or 13, wherein the treatment increases or extends the survival of a subject having a tumour.

15. A biomolecule conjugate according to any one of claims 1 to 5 or pharmaceutical composition according to claim 6 or 7 for use in a method for increasing the sensitivity of fibrotic tissue or atherosclerotic tissue to an anti-fibrotic agent or anti-atherosclerotic agent.

16. A biomolecule conjugate or pharmaceutical composition for use according to claim 12, wherein treating atherosclerosis comprises treating or inhibiting the formation of atherosclerotic plaque formation, increasing plasma HDL levels and/or decreasing plasma LDL levels.

17. A biomolecule conjugate according to any one of claims 1 to 5 or pharmaceutical composition according to claim 6 or 7 for use in a method for identifying, imaging or localizing cancerous cells and tumours in a subject, wherein the method comprises administering to the subject the biomolecule conjugate or pharmaceutical composition in combination with a tumour or cancer cell imaging agent.

18. A biomolecule conjugate according to any one of claims 1 to 5 or pharmaceutical composition according to claim 6 or 7 for use in a method for identifying, imaging or localizing fibrotic tissue in a subject, wherein the method comprises administering to the subject the biomolecule conjugate or pharmaceutical composition in combination with an agent for visualising fibrotic tissue.

19. A peptide comprising or consisting of the sequence set forth in SEQ ID NO:1 for use in a method for detecting and/or localising atherosclerotic or fibrotic tissue.

## Patentansprüche

1. Biomolekülkonjugat, umfassend ein Zytokin und ein Peptid, umfassend oder bestehend aus der Sequenz, die in SEQ ID NR:1 angegeben ist, oder einer konservativen Variante davon.

2. Biomolekülkonjugat nach Anspruch 1, wobei das Zytokin ein immunverstärkendes Zytokin ist, das eine zelluläre Immunantwort vermittelt.

3. Biomolekülkonjugat nach Anspruch 2, wobei das immunverstärkende Zytokin TNFα oder IFNγ ist.

4. Biomolekülkonjugat nach einem der Ansprüche 1 bis 3, wobei das Peptid, das die Sequenz, die in SEQ ID NR:1 angegeben ist, oder eine konservative Variante davon umfasst oder daraus besteht, an das C-endständige Ende des Zytokins konjugiert ist.

5. Biomolekülkonjugat nach Anspruch 4, wobei das Peptid an das C-endständige Ende des Zytokins über eine Linkersequenz konjugiert ist, die einen oder mehr, zwei oder mehr oder drei oder mehr Glycin- (G) Reste umfasst.

6. Pharmazeutische Zusammensetzung, umfassend ein Biomolekülkonjugat nach einem der Ansprüche 1 bis 3 oder ein Polynukleotid, das für dieses codiert, wobei die Zusammensetzung weiter einen oder mehrere pharmazeutisch annehmbare Träger, Adjuvantien und/oder Hilfsstoffe umfasst.

7. Pharmazeutische Zusammensetzung nach Anspruch 6, weiter umfassend ein oder mehrere zusätzliche Anti-Tumor-, anti-atherosklerotische oder anti-fibrotische Mittel.

8. Biomolekülkonjugat nach einem der Ansprüche 1 bis 5 oder pharmazeutische Zusammensetzung nach Anspruch 6 oder 7 zur Verwendung in einem Verfahren zur Behandlung eines Zustands, der mit abnormaler ECM verbunden ist.

9. Biomolekülkonjugat nach einem der Ansprüche 1 bis 5 oder pharmazeutische Zusammensetzung nach Anspruch 6 oder 7 zur Verwendung nach Anspruch 8, wobei die Behandlung die extrazelluläre Matrix (ECM) von Tumor, atherosklerotischem oder fibrotischem Gewebe abbaut oder Immunzellinfiltration eines Tumors, atherosklerotischen Gewebes oder fibrotischen Gewebes fördert oder auslöst.

10. Biomolekülkonjugat oder pharmazeutische Zusammensetzung zur Verwendung nach Anspruch 9, wobei die Immunzellen, die den Tumor oder das Gewebe infiltrieren, eine oder mehrere Proteasen exprimieren oder freisetzen, die imstande sind, Tumor-ECM abzubauen.

11. Biomolekülkonjugat oder pharmazeutische Zusammensetzung zur Verwendung nach Anspruch 9 oder 10, wobei die Immunzellen T-Zellen, Makrophagen und/oder Neutrophile umfassen.

12. Biomolekülkonjugat oder pharmazeutische Zusammensetzung zur Verwendung nach Anspruch 8, wobei der Zustand ein solider Tumor, Atherosklerose oder Fibrose ist.

13. Biomolekülkonjugat oder pharmazeutische Zusammensetzung zur Verwendung nach Anspruch 12, wobei Behandlung des Tumors mit dem Konjugat oder der Zusammensetzung Gefäßperfusion in dem Tumor erhöht, Zugang des einen oder der mehreren zusätzlichen Anti-Krebsmittel zu den Tumor- und kanzerösen Zellen darin erhöht und dadurch Wirksamkeit der Anti-Krebsmittel erhöht.

14. Biomolekülkonjugat oder pharmazeutische Zusammensetzung zur Verwendung nach Anspruch 12 oder 13, wobei die Behandlung das Überleben eines Subjekts mit einem Tumor erhöht oder verlängert.

15. Biomolekülkonjugat nach einem der Ansprüche 1 bis 5 oder pharmazeutische Zusammensetzung nach Anspruch 6 oder 7 zur Verwendung in einem Verfahren zur Erhöhung der Empfindlichkeit von fibrotischem Gewebe oder atherosklerotischem Gewebe für ein anti-fibrotisches Mittel oder anti-atherosklerotisches Mittel.

16. Biomolekülkonjugat oder pharmazeutische Zusammensetzung zur Verwendung nach Anspruch 12, wobei Behandlung von Atherosklerose Behandeln oder Verhindern der Bildung von atherosklerotischer Plaquebildung, Erhöhen von Plasma-HDL-Spiegeln und/oder Senken von Plasma-LDL-Spiegeln umfasst.

17. Biomolekülkonjugat nach einem der Ansprüche 1 bis 5 oder pharmazeutische Zusammensetzung nach Anspruch 6 oder 7 zur Verwendung in einem Verfahren zum Identifizieren, Abbilden oder Lokalisieren kanzeröser Zellen und Tumore in einem Subjekt, wobei das Verfahren Verabreichen des Biomolekülkonjugats oder der pharmazeutischen Zusammensetzung an das Subjekt in Kombination mit einem Tumor- oder Krebszellenbildgebungsmittel umfasst.

18. Biomolekülkonjugat nach einem der Ansprüche 1 bis 5 oder pharmazeutische Zusammensetzung nach Anspruch 6 oder 7 zur Verwendung in einem Verfahren zum Identifizieren, Abbilden oder Lokalisieren fibrotischen Gewebes in einem Subjekt, wobei das Verfahren Verabreichen des Biomolekülkonjugats oder der pharmazeutischen Zusammensetzung an das Subjekt in Kombination mit einem Mittel zum Sichtbarmachen von fibrotischem Gewebe umfasst.

19. Peptid, umfassend die oder bestehend aus der Sequenz, die in SEQ ID NR:1 angegeben ist, zur Verwendung in einem Verfahren zum Nachweisen und/oder Lokalisieren von atherosklerotischem oder fibrotischem Gewebe.

## Revendications

1. Conjugué biomoléculaire comprenant une cytokine et un peptide comprenant ou consistant en la séquence représentée par SEQ ID NO : 1 ou un variant conservatif de celle-ci.

2. Conjugué biomoléculaire selon la revendication 1, dans lequel la cytokine est une cytokine d'immunopotentialisation qui médie une réponse immunitaire cellulaire.

3. Conjugué biomoléculaire selon la revendication 2, dans lequel la cytokine d'immunopotentialisation est TNFα ou IFNγ.

4. Conjugué biomoléculaire selon l'une quelconque des revendications 1 à 3, dans lequel le peptide comprenant ou consistant en la séquence représentée par SEQ ID NO : 1, ou un variant conservatif de celui-ci, est conjugué à l'extrémité C-terminale de la cytokine.

5. Conjugué biomoléculaire selon la revendication 4, dans lequel le peptide est conjugué à l'extrémité C-terminale de la cytokine via une séquence de lieur comprenant un ou plus, deux ou plus, ou trois ou plus, résidus de glycine (G).

6. Composition pharmaceutique comprenant un conjugué biomoléculaire selon l'une quelconque des revendications 1 à 3, ou un polynucléotide codant pour celui-ci, dans laquelle la composition comprend en outre un ou plusieurs supports, adjuvants et/ou excipients pharmaceutiquement acceptables.

7. Composition pharmaceutique selon la revendication 6, comprenant en outre un ou plusieurs agents anti-tumorigènes, anti-athéroscléreux ou anti-fibrogènes supplémentaires.

8. Conjugué biomoléculaire selon l'une quelconque des revendications 1 à 5 ou composition pharmaceutique selon la revendication 6 ou 7 pour une utilisation dans un procédé de traitement d'une affection associée à une MEC anormale.

9. Conjugué biomoléculaire selon l'une quelconque des revendications 1 à 5 ou composition pharmaceutique selon la revendication 6 ou 7 pour une utilisation selon la revendication 8, dans lequel le traitement dégrade la matrice extracellulaire (MEC) d'un tissu tumoral, athéroscléreux ou fibreux ou favorise ou induit l'infiltration d'une tumeur, d'un tissu athéroscléreux ou d'un tissu fibreux par des cellules immunitaires.

10. Conjugué biomoléculaire ou composition pharmaceutique pour une utilisation selon la revendication 9, dans lequel les cellules immunitaires infiltrant la tumeur ou le tissu expriment ou libèrent une ou plusieurs protéases capables de dégrader la MEC tumorale.

11. Conjugué biomoléculaire ou composition pharmaceutique pour une utilisation selon la revendication 9 ou 10, dans lequel les cellules immunitaires comprennent des cellules T, des macrophages et/ou des neutrophiles.

12. Conjugué biomoléculaire ou composition pharmaceutique pour une utilisation selon la revendication 8, dans lequel l'affection est une tumeur solide, une athérosclérose ou une fibrose.

13. Conjugué biomoléculaire ou composition pharmaceutique pour une utilisation selon la revendication 12, dans lequel le traitement de la tumeur avec le conjugué ou la composition augmente la perfusion des vaisseaux dans la tumeur, en augmentant l'accès des un ou plusieurs agents anticancéreux supplémentaires à la tumeur et aux cellules cancéreuses à l'intérieur de celle-ci et en améliorant ainsi l'efficacité desdits agents anticancéreux.

14. Conjugué biomoléculaire ou composition pharmaceutique pour une utilisation selon la revendication 12 ou 13, dans lequel le traitement augmente ou prolonge la survie d'un sujet présentant une tumeur.

15. Conjugué biomoléculaire selon l'une quelconque des revendications 1 à 5 ou composition pharmaceutique selon la revendication 6 ou 7 pour une utilisation dans un procédé d'augmentation de la sensibilité d'un tissu fibreux ou d'un tissu athéroscléreux à un agent anti-fibrogène ou un agent anti-athéroscléreux.

16. Conjugué biomoléculaire ou composition pharmaceutique pour une utilisation selon la revendication 12, dans lequel le traitement d'une athérosclérose comprend le traitement ou l'inhibition de la formation de plaques athéroscléreuses, l'augmentation des taux plasmatiques de HDL et/ou la diminution des taux plasmatiques de LDL.

17. Conjugué biomoléculaire selon l'une quelconque des revendications 1 à 5 ou composition pharmaceutique selon la revendication 6 ou 7 pour une utilisation dans un procédé d'identification, d'imagerie ou de localisation de cellules cancéreuses et de tumeurs chez un sujet, dans lequel le procédé comprend l'administration au sujet du conjugué biomoléculaire ou de la composition pharmaceutique en combinaison avec un agent d'imagerie de tumeurs ou de cellules cancéreuses.

18. Conjugué biomoléculaire selon l'une quelconque des revendications 1 à 5 ou composition pharmaceutique selon la revendication 6 ou 7 pour une utilisation dans un procédé d'identification, d'imagerie ou de localisation d'un tissu fibreux chez un sujet, dans lequel le procédé comprend l'administration au sujet du conjugué biomoléculaire ou de la composition pharmaceutique en combinaison avec un agent pour visualiser un tissu fibreux.

19. Peptide comprenant ou consistant en la séquence représentée par SEQ ID NO : 1 pour une utilisation dans un procédé de détection et/ou de localisation d'un tissu athéroscléreux ou fibreux.


### SEQUENCE LISTING

<110> The University of Western Australia
<120> Novel Biomolecule Conjugates And Uses Therefor
<130> 35261131
<160> 10
<170> PatentIn version 3.5
<210> 1
   <211> 9
   <212> PRT
   <213> Artificial Sequence
<220>
   <223> Synthetic sequence
<400> 1
   Cys Ser Gly Arg Arg Ser Ser Lys Cys
   1               5
<210> 2
   <211> 233
   <212> PRT
   <213> Homo sapiens
<400> 2
   Met Ser Thr Glu Ser Met Ile Arg Asp Val Glu Leu Ala Glu Glu Ala
   1               5                   10                  15
   Leu Pro Lys Lys Thr Gly Gly Pro Gln Gly Ser Arg Arg Cys Leu Phe
               20                  25                  30
   Leu Ser Leu Phe Ser Phe Leu Ile Val Ala Gly Ala Thr Thr Leu Phe
           35                  40                  45
   Cys Leu Leu His Phe Gly Val Ile Gly Pro Gln Arg Glu Glu Phe Pro
       50                  55                  60
   Arg Asp Leu Ser Leu Ile Ser Pro Leu Ala Gln Ala Val Arg Ser Ser
   65                  70                  75                  80
   Ser Arg Thr Pro Ser Asp Lys Pro Val Ala His Val Val Ala Asn Pro
                   85                  90                  95
   Gln Ala Glu Gly Gln Leu Gln Trp Leu Asn Arg Arg Ala Asn Ala Leu
               100                 105                 110
   Leu Ala Asn Gly Val Glu Leu Arg Asp Asn Gln Leu Val Val Pro Ser
           115                 120                 125
   Glu Gly Leu Tyr Leu Ile Tyr Ser Gln Val Leu Phe Lys Gly Gln Gly
       130                 135                 140
   Cys Pro Ser Thr His Val Leu Leu Thr His Thr Ile Ser Arg Ile Ala
   145                 150                 155                 160
   Val Ser Tyr Gln Thr Lys Val Asn Leu Leu Ser Ala Ile Lys Ser Pro
                   165                 170                 175
   Cys Gln Arg Glu Thr Pro Glu Gly Ala Glu Ala Lys Pro Trp Tyr Glu
               180                 185                 190
   Pro Ile Tyr Leu Gly Gly Val Phe Gln Leu Glu Lys Gly Asp Arg Leu
           195                 200                 205
   Ser Ala Glu Ile Asn Arg Pro Asp Tyr Leu Asp Phe Ala Glu Ser Gly
       210                 215                 220
   Gln Val Tyr Phe Gly Ile Ile Ala Leu
   225                 230
<210> 3
   <211> 157
   <212> PRT
   <213> Homo sapiens
<400> 3
<210> 4
   <211> 169
   <212> **PRT**
   <213> **Artificial** Sequence
<220>
   <223> Synthetic sequence
<400> 4
   Val Arg Ser Ser Ser Arg Thr Pro Ser Asp Lys Pro Val Ala His Val
   1               5                   10                  15
   Val Ala Asn Pro Gln Ala Glu Gly Gln Leu Gln Trp Leu Asn Arg Arg
               20                  25                  30
   Ala Asn Ala Leu Leu Ala Asn Gly Val Glu Leu Arg Asp Asn Gln Leu
           35                  40                  45
   Val Val Pro Ser Glu Gly Leu Tyr Leu Ile Tyr Ser Gln Val Leu Phe
       50                  55                  60
   Lys Gly Gln Gly Cys Pro Ser Thr His Val Leu Leu Thr His Thr Ile
   65                  70                  75                  80
   Ser Arg Ile Ala Val Ser Tyr Gln Thr Lys Val Asn Leu Leu Ser Ala
                   85                  90                  95
   Ile Lys Ser Pro Cys Gln Arg Glu Thr Pro Glu Gly Ala Glu Ala Lys
               100                 105                 110
   Pro Trp Tyr Glu Pro Ile Tyr Leu Gly Gly Val Phe Gln Leu Glu Lys
           115                 120                 125
   Gly Asp Arg Leu Ser Ala Glu Ile Asn Arg Pro Asp Tyr Leu Asp Phe
       130                 135                 140
   Ala Glu Ser Gly Gln Val Tyr Phe Gly Ile Ile Ala Leu Gly Gly Gly
   145                 150                 155                 160
   Cys Ser Gly Arg Arg Ser Ser Lys Cys
                           165
<210> 5
   <211> 10
   <212> PRT
   <213> Artificial Sequence
<220>
   <223> Synthetic sequence
<400> 5
   Ala Arg Ala Leu Pro Ser Gln Arg Ser Arg
   1               5                   10
<210> 6
   <211> 156
   <212> **PRT**
   <213> Mus musculus
<400> 6
   Leu Arg Ser Ser Ser Gln Asn Ser Ser Asp Lys Pro Val Ala His Val
   1               5                   10                  15
   Val Ala Asn His Gln Val Glu Glu Gln Leu Glu Trp Leu Ser Gln Arg
               20                  25                  30
   Ala Asn Ala Leu Leu Ala Asn Gly Met Asp Leu Lys Asp Asn Gln Leu
           35                  40                  45
   Val Val Pro Ala Asp Gly Leu Tyr Leu Val Tyr Ser Gln Val Leu Phe
       50                  55                  60
   Lys Gly Gln Gly Cys Pro Asp Tyr Val Leu Leu Thr His Thr Val Ser
   65                  70                  75                  80
   Arg Phe Ala Ile Ser Tyr Gln Glu Lys Val Asn Leu Leu Ser Ala Val
                   85                  90                  95
   Lys Ser Pro Cys Pro Lys Asp Thr Pro Glu Gly Ala Glu Leu Lys Pro
               100                 105                 110
   Trp Tyr Glu Pro Ile Tyr Leu Gly Gly Val Phe Gln Leu Glu Lys Gly
           115                 120                 125
   Asp Gln Leu Ser Ala Glu Val Asn Leu Pro Lys Tyr Leu Asp Phe Ala
       130                 135                 140
   Glu Ser Gly Gln Val Tyr Phe Gly Val Ile Ala Leu
   145 150 155
<210> 7
   <211> 166
   <212> **PRT**
   <213> Homo sapiens
<400> 7
   Met Lys Tyr Thr Ser Tyr Ile Leu Ala Phe Gln Leu Cys Ile Val Leu
   1               5                   10                  15
   Gly Ser Leu Gly Cys Tyr Cys Gln Asp Pro Tyr Val Lys Glu Ala Glu
               20                  25                  30
   Asn Leu Lys Lys Tyr Phe Asn Ala Gly His Ser Asp Val Ala Asp Asn
           35                  40                  45
   Gly Thr Leu Phe Leu Gly Ile Leu Lys Asn Trp Lys Glu Glu Ser Asp
       50                  55                  60
   Arg Lys Ile Met Gln Ser Gln Ile Val Ser Phe Tyr Phe Lys Leu Phe
   65                  70                  75                  80
   Lys Asn Phe Lys Asp Asp Gln Ser Ile Gln Lys Ser Val Glu Thr Ile
                   85                  90                  95
   Lys Glu Asp Met Asn Val Lys Phe Phe Asn Ser Asn Lys Lys Lys Arg
               100                 105                 110
   Asp Asp Phe Glu Lys Leu Thr Asn Tyr Ser Val Thr Asp Leu Asn Val
           115                 120                 125
   Gln Arg Lys Ala Ile His Glu Leu Ile Gln Val Met Ala Glu Leu Ser
       130                 135                 140
   Pro Ala Ala Lys Thr Gly Lys Arg Lys Arg Ser Gln Met Leu Phe Arg
   145                 150                 155                 160
   Gly Arg Arg Ala Ser Gln
                   165
<210> 8
   <211> 143
   <212> **PRT**
   <213> Homo sapiens
<400> 8
   Gln Asp Pro Tyr Val Lys Glu Ala Glu Asn Leu Lys Lys Tyr Phe Asn
   1               5                   10                  15
   Ala Gly His Ser Asp Val Ala Asp Asn Gly Thr Leu Phe Leu Gly Ile
               20                  25                  30
   Leu Lys Asn Trp Lys Glu Glu Ser Asp Arg Lys Ile Met Gln Ser Gln
           35                  40                  45
   Ile Val Ser Phe Tyr Phe Lys Leu Phe Lys Asn Phe Lys Asp Asp Gln
       50                  55                  60
   Ser Ile Gln Lys Ser Val Glu Thr Ile Lys Glu Asp Met Asn Val Lys
   65                  70                  75                  80
   Phe Phe Asn Ser Asn Lys Lys Lys Arg Asp Asp Phe Glu Lys Leu Thr
                   85                  90                  95
   Asn Tyr Ser Val Thr Asp Leu Asn Val Gln Arg Lys Ala Ile His Glu
               100                 105                 110
   Leu Ile Gln Val Met Ala Glu Leu Ser Pro Ala Ala Lys Thr Gly Lys
           115                 120                 125
   Arg Lys Arg Ser Gln Met Leu Phe Arg Gly Arg Arg Ala Ser Gln
       130                 135                 140
<210> 9
   <211> 155
   <212> PRT
   <213> Artificial Sequence
<220>
   <223> Synthetic sequence
<400> 9
   Gln Asp Pro Tyr Val Lys Glu Ala Glu Asn Leu Lys Lys Tyr Phe Asn
   1               5                   10                  15
   Ala Gly His Ser Asp Val Ala Asp Asn Gly Thr Leu Phe Leu Gly Ile
               20                  25                  30
   Leu Lys Asn Trp Lys Glu Glu Ser Asp Arg Lys Ile Met Gln Ser Gln
           35                  40                  45
   Ile Val Ser Phe Tyr Phe Lys Leu Phe Lys Asn Phe Lys Asp Asp Gln
       50                  55                  60
   Ser Ile Gln Lys Ser Val Glu Thr Ile Lys Glu Asp Met Asn Val Lys
   65                  70                  75                  80
   Phe Phe Asn Ser Asn Lys Lys Lys Arg Asp Asp Phe Glu Lys Leu Thr
                   85                  90                  95
   Asn Tyr Ser Val Thr Asp Leu Asn Val Gln Arg Lys Ala Ile His Glu
               100                 105                 110
   Leu Ile Gln Val Met Ala Glu Leu Ser Pro Ala Ala Lys Thr Gly Lys
           115                 120                 125
   Arg Lys Arg Ser Gln Met Leu Phe Arg Gly Arg Arg Ala Ser Gln Gly
       130                 135                 140
   Gly Gly Cys Ser Gly Arg Arg Ser Ser Lys Cys
   145                 150                 155
<210> 10
   <211> 133
   <212> PRT
   <213> Mus musculus
<400> 10
   His Gly Thr Val Ile Glu Ser Leu Glu Ser Leu Asn Asn Tyr Phe Asn
   1               5                   10                  15
   Ser Ser Gly Ile Asp Val Glu Glu Lys Ser Leu Phe Leu Asp Ile Trp
               20                  25                  30
   Arg Asn Trp Gln Lys Asp Gly Asp Met Lys Ile Leu Gln Ser Gln Ile
           35                  40                  45
   Ile Ser Phe Tyr Leu Arg Leu Phe Glu Val Leu Lys Asp Asn Gln Ala
       50                  55                  60
   Ile Ser Asn Asn Ile Ser Val Ile Glu Ser His Leu Ile Thr Thr Phe
   65                  70                  75                  80
   Phe Ser Asn Ser Lys Ala Lys Lys Asp Ala Phe Met Ser Ile Ala Lys
                   85                  90                  95
   Phe Glu Val Asn Asn Pro Gln Val Gln Arg Gln Ala Phe Asn Glu Leu
               100                 105                 110
   Ile Arg Val Val His Gln Leu Leu Pro Glu Ser Ser Leu Arg Lys Arg
           115                 120                 125
   Lys Arg Ser Arg Cys
       130